# EUROPEAN PATENT SPECIFICATION

(11) **EP 2 007 753 B1**
(45) Date of publication and mention of the grant of the patent: **11.11.2009**
(21) Application number: 07727623.6
(22) Date of filing: 02.04.2007
(51) Int. Cl.: C07D 403/06, C07D 403/04, C07D 413/04, C07D 413/06, A61K 31/416, A61P 25/22, A61P 25/24

(54) **HETEROCYCLIC GABA ALPHA SUBTYPE SELECTIVE RECEPTOR MODULATORS**
HETEROCYCLISCHE MODULATOREN FÜR DEN GABA-ALPHA-SUBTYP-SELEKTIVEN REZEPTOR
MODULATEURS DU RÉCEPTEUR SÉLECTIF DU SOUS-TYPE DE GABA ALPHA HÉTÉROCYCLIQUE

(30) Priority: 11.04.2006 US 791264 P
(43) Date of publication of application: 31.12.2008
(73) Proprietor: F. Hoffmann-La Roche AG, 4070 Basel (CH)
(72) Inventor: LIN, Xiao-Fa, Palo Alto, California 94303 (US); LOUGHHEAD, David Garrett, Belmont, California 94002 (US); MANKA, Jason, Sunnyvale, California 94086 (US); O'YANG, Counde, Sunnyvale, California 94087 (US); SOTH, Michael, Milpitas, California 95035 (US); YASUDA, Dennis Mitsugu, Campbell, California 95008 (US)
(74) Representative: Wasner, Marita
(86) International application number: PCT/EP2007/053151
(87) International publication number: WO 2007/115966

(56) References cited:
- WO-A-2005/016892
- US-A1- 2005 101 614

## Description

This invention relates to novel substituted 7-arylindazole of formula I, the use of such compounds for modulating α₂ subtype GABA_{A} receptors and pharmaceutical compositions containing such compounds of formula I 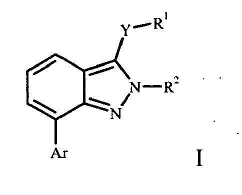 wherein:
- Y: is (CH₂)ₙ, alkenylene, CH(OR³) or C=O;
- R¹: is a five-membered heteroaryl containing at least one nitrogen ring atom, wherein said five-membered heteroaryl ring is optionally substituted with one or more substituents each of which is independently selected from the group consisting of C₁₋₆ alkyl, C₁₋₆ heteroalkyl, -(CH₂)ₘCOX¹, -(CH₂)ₘSO₂X² and oxo;
- X¹: is-OR³ or-NR⁴R⁵;
- X²: is C₁₋₆ alkyle or -NR⁴R⁵;
- R²: is hydrogen, C₁₋₆ alkyl, C₁₋₆ haloalkyl or C₃-₆cycloalkyl; .
- Ar: is aryl or heteroaryl, each optionally substituted with one or more substituents independently selected from the group consisting of C₁₋₆ alkyl, C₁₋₆ alkoxy, C₁₋₆ alkylthio, C₁₋₆ alkylsulfonyl, -SO₂NR⁴R⁵, halogen, C₁₋₆ haloalkyl, cyano, nitro, and -NR⁶R7;
- each of R⁶ and R⁷: is independently selected from the group consisting of hydrogen, C₁₋₉ alkyl, and C₁₋₉ alkylcarbonyl;
- each of R³, R⁴, and R⁵: is independently hydrogen or C₁₋₆ alkyl;
- m: is an integer from 0 to 4; and
- n: is an integer from 0 to 3.
and pharmaceutically acceptable salts thereof.

4-Aminobutyric acid (γ-aminobutyric acid or GABA) is the primary inhibitory transmitter in the brain and maintains a balance between excitation and inhibition of neurons. Three major classes of GABA receptors have been identified: ABA_{A}, ABA_{B} and GABA_{C} receptors. GABA_{A} and GABA_{C} receptors are ligand-gated ion channels (LGIC), while GABA_{B} receptors are G-protein coupled receptors. The LGIC receptors are heteropentamers comprised of α₁₋₆, β₁₋₃, γ₁₋₃, ρ₁₋₃, δ, ε, π, and θ subunits. Each subunit contains four membrane-spanning domains. The N-terminal domain and C-domain are extracellular and the agonist/antagonist binding site is situated on the N-terminus. There is an intracellular loop between the 3 ^{rd} and 4^{th} membrane spanning regions (M. Chabib and G.A.R. Johnston, J. Med. Chem. 2000 43(8):1427-1447).

While studies are continuing to define the composition and anatomical distribution of GABA LGIC receptors, it is known that the dominant motif is 2α2βγ with varying α subtypes. Subtype assemblies containing an α₁ subunit are present in most areas of the brain and are thought to account for over 40% of GABA_{A} receptors in the rat. Subtype assemblies containing α₂β_{2/3}γ₂ and α₃βₙγ_{2/3} oligomers are thought to account for about 18% and 17% respectively of GABA_{A} receptors in the rat (R.M. McKernan et al. Trend Neurosci 1996 19:139-143). Subtype assemblies containing an α₅ subunit are expressed predominantly in the hippocampus and cortex and are thought to represent about 4% of GABA_{A} receptors in the rat. The most common receptor subtype assemblies appear to be the α₁β₂γ₂, α₂β₃γ₂, α₃β₃γ₂ and α₅β₃γ₂ assemblies (H. Mohler et al. Neuroch. Res. 1995 20(5):631-636).

All known GABA_{A} receptors contain a plurality of distinct modulatory sites, one of which is the benzodiazepine (BZ) binding site. Other modulatory sites include allosteric sites for picrotoxin, barbiturates, neuroactive steroids and ethanol. The BZ binding site is the most explored of the GABA_{A} receptor modulatory sites, and is the site through which anxiolytic drugs such as diazepam exert their effect. Early radioligand binding studies suggested the existence of two distinct benzodiazepine-binding sites: BZ1 and BZ2. The BZ1 subtype has been shown to be pharmacologically equivalent to a GABA_{A} receptor comprising the α₁ subunit in combination with a β subunit and γ₂. This is the most abundant GABA_{A} receptor subtype. Two other major populations are the α₂βγ₂ and α₃β_{2/3}γ₂ subtypes. Together these constitute approximately a further 35% of the total GABA_{A} receptor repertoire. Pharmacologically, the α₂βγ₂ and α₃β_{2/3}γ₂ subtypes appear to be equivalent to the BZ2 subtype. The physiological role of these subtypes has hitherto been unclear because sufficiently selective agonists or antagonists were unknown.

The barbiturates and benzodiazepines were among the first clinically useful modulators of the GABA receptors and are among the most widely prescribed medications for anxiety, depression and other psychiatric disorders and as anticonvulsants. Benzodiazepines, with relatively mild side effects, afforded an alternative to barbiturates which possess more potent side effects. Unfortunately, many of the early benzodiazepines had relative limited subtype selectivity resulting in sedation, dependence, cognitive impairment, ataxia, potentiation of ethanol effects, tolerance and withdrawal.

The advances in genetics and molecular biology have afforded more subtle probes of receptor subtype selectivity and hold out the promise of more selective agents. Receptors containing the α₁, α₂, α₃ or α₅ subunit have been classified as diazepam sensitive receptors while α₄, or α₆, are classified as diazepam insensitive receptors. In particular, the α₁ subtype has been associated with sedation and α₁ selective ligands have potential as sedatives (R.M. McKernan et al. Nature Neurosci. 2000 3(6): 587-592). Hypnotic/ sedative compounds with preferential binding for the α₁ subtype have been identified (D. J. Sanger and H. Depoortere, CNS Drug Reviews, 1998 47(5):323-340). Sedation, however, is undesirable in an anxiolytic agent.

Compounds that selectively bind to the benzodiazepine site, or to other allosteric sites, and enhance the ability of GABA to open GABA_{A} receptor channels are agonists (or positive allosteric modulators) of GABA receptors. Compounds that interact with allosteric sites but negatively modulate the action of GABA are called inverse agonists (negative allosteric modulators). Inverse agonists diminish the ability of GABA to open receptor channels. A third class of compounds that bind selectively to the benzodiazepine site and yet have little or no effect on GABA activity, but can block the action of GABA_{A} receptor agonists or inverse agonists that act at this site are referred to as antagonists. Agonists that act at the benzodiazepine site exhibit anxiolytic, sedative, and hypnotic effects, while compounds that act as inverse agonists at this site elicit anxiogenic, cognition enhancing, and proconvulsant effects.

The α₁ selective GABA_{A} receptor agonists alpidem and zolpidem are clinically prescribed as hypnotic agents, further suggesting that at least some of the sedation associated with known anxiolytic drugs is mediated through GABA_{A} receptors containing the α₁ subunit. Accordingly, GABA_{A} receptor agonists which interact more selectively with the α₂ and/or the α₃ subunit relative to the α₁ subunit should retain anxiolytic activity with a reduced propensity to cause sedation. Also, agents which are antagonists or inverse agonists at the α₁ subtype might antagonize sedation or hypnosis caused by α₁ modulators.

Selective α₂ and α₃ ligands have been more difficult to identify and cross-reactivity between these receptors is common. Compounds with ten to one hundred-fold selectivity for α_{2/3} relative to α₁ have been reported (see, *e.g.,* W. R. Carling et al., WO 0044752). Experiments with point mutated mice lines suggest that the α₂, not the α₃, subtype is responsible for the anxiolytic activity (U. Rudolph et al. Trends Pharmacol. Sci. 2001 22(4):188-194; K. Löw et al. Science 2000 290:131-134); however, α₃-selective inverse agonists appear to be anxiogenic and proconvulsant ( I. J. Collins et al. WO 9855480). Since α₂ and perhaps α₃ and α₅ selective ligands have the potential to modulate the (BZ2) site without activating the hypnotic sedative site (BZ1) they could afford a new class of non-sedating anxiolytics. Other non-BZ selective α₂ GABA modulators may also exhibit anxiolytic properties without many of unwanted effects.

The α₅ subtype is located predominantly in the hippocampus and thought to be associated with learning and cognition. Approximately 20% of the hippocampal GABA receptors contain the α₅ subtype. The hippocampus is the region of the brain associated with learning and memory. Inverse α₅ agonists improve memory and cognition in animal behavioral models; however, their use is limited by the anxiogenic and convulsant effects of non selective compounds. Selective α₅ inverse agonists are potentially useful in the treatment Alzheimer's disease and related dementias (M. S. Chambers et al., J. Med. Chem. 2003 46(11):2227-40). Further indications may be found in WO2005/016892 and US 2005/101614.

Therefore, there is a need for selective ligands for GABA_{A} receptors.

The invention also provides pharmaceutical compositions, methods of using, and methods of preparing the aforementioned compounds.

Compounds and compositions of the present invention are useful in the treatment and/or prevention of anxiety disorders, such as panic disorder with or without agoraphobia, agoraphobia without history of panic disorder, phobias including social phobias, obsessive-compulsive disorder, stress disorders including post-traumatic and acute stress disorder, and generalized or substance-induced anxiety disorder; depression or bipolar disorders such as single-episode or recurrent major depressive disorder, dysthymic disorder, bipolar I and bipolar II manic disorders; schizophrenia; learning and cognitive disorder such as Alzheimer's disease and attention deficit hyperactivity disorder; sleep disorders and disorders of circadian rhythm, e.g. in subjects suffering from the effects of jet lag or shift work; convulsive or seizure disorders such as epilepsy and pain.

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

"Alkyl" means the monovalent linear or branched saturated hydrocarbon moiety, consisting solely of carbon and hydrogen atoms, having from one to twelve carbon atoms. "Lower alkyl" refers to an alkyl group of one to six carbon atoms, i.e. C₁-C₆alkyl. Examples of alkyl groups include, but are not limited to, methyl, ethyl, propyl, isopropyl, isobutyl, sec-butyl, tert-butyl, pentyl, n-hexyl, octyl, dodecyl, and the like. "Branched alkyl" means isopropyl, isobutyl, tert-butyl.

"Alkylene" means a linear saturated divalent hydrocarbon radical of one to six carbon atoms or a branched saturated divalent hydrocarbon radical of three to six carbon atoms, e.g., methylene, ethylene, 2,2-dimethylethylene, propylene, 2-methylpropylene, butylene, pentylene, and the like.

"Aryl" means a monovalent cyclic aromatic hydrocarbon moiety consisting of a mono-, bi- or tricyclic aromatic ring. The aryl group can be optionally substituted as defined herein. Examples of aryl moieties include, but are not limited to, optionally substituted phenyl, naphthyl, phenanthryl, fluorenyl, indenyl, pentalenyl, azulenyl, oxydiphenyl, biphenyl, methylenediphenyl, aminodiphenyl, diphenylsulfidyl, diphenylsulfonyl, diphenylisopropylidenyl, benzodioxanyl, benzofuranyl, benzodioxylyl, benzopyranyl, benzoxazinyl, benzoxazinonyl, benzopiperadinyl, benzopiperazinyl, benzopyrrolidinyl, benzomorpholinyl, methylenedioxyphenyl, ethylenedioxyphenyl, and the like, including partially hydrogenated derivatives thereof.

"Cycloalkyl" means a monovalent saturated carbocyclic moiety consisting of mono- or bicyclic rings. Cycloalkyl can optionally be substituted with one or more substituents, wherein each substituent is independently hydroxy, alkyl, alkoxy, halo, haloalkyl, amino, monoalkylamino, or dialkylamino, unless otherwise specifically indicated. Examples of cycloalkyl moieties include, but are not limited to, cyclopropyl, cyclobutyl, cyclopentyl, cyclohexyl, cycloheptyl, and the like, including partially unsaturated derivatives thereof.

"Heteroalkyl" means an alkyl radical as defined herein, including a branched C₄-C₇-alkyl, wherein one, two or three hydrogen atoms have been replaced with a substituent independently selected from the group consisting of -OR^{a}, -NR^{b}R^{c}, and -S(O)ₙR^{d} (where n is an integer from 0 to 2), with the understanding that the point of attachment of the heteroalkyl radical is through a carbon atom, wherein R^{a} is hydrogen, acyl, alkyl, cycloalkyl, or cycloalkylalkyl; R^{b} and R^{c} are independently of each other hydrogen, acyl, alkyl, cycloalkyl, or cycloalkylalkyl; and when n is 0, R^{d} is hydrogen, alkyl, cycloalkyl, or cycloalkylalkyl, and when n is 1 or 2; R^{d} is alkyl, cycloalkyl, cycloalkylalkyl, amino, acylamino, monoalkylamino, or dialkylamino. Representative examples include, but are not limited to, 2-hydroxyethyl, 3-hydroxypropyl, 2-hydroxy-1-hydroxymethylethyl, 2,3-dihydroxypropyl, 1-hydroxymethylethyl, 3-hydroxybutyl, 2,3-dihydroxybutyl, 2-hydroxy-1-methylpropyl, 2-aminoethyl, 3-aminopropyl, 2-methylsulfonylethyl, aminosulfonylmethyl, aminosulfonylethyl, aminosulfonylpropyl, methylaminosulfonylmethyl, methylaminosulfonylethyl, methylaminosulfonylpropyl, and the like.

"Heteroaryl" means a monocyclic or bicyclic radical of 5 to 12 ring atoms having at least one aromatic ring containing one, two, or three ring heteroatoms selected from N, O, or S, the remaining ring atoms being C, with the understanding that the attachment point of the heteroaryl radical will be on an aromatic ring. The heteroaryl ring may be optionally substituted as defined herein. Examples of heteroaryl moieties include, but are not limited to, optionally substituted imidazolyl, oxazolyl, isoxazolyl, thiazolyl, isothiazolyl, oxadiazolyl, thiadiazolyl, pyrazinyl, thienyl, thiophenyl, furanyl, pyranyl, pyridinyl, pyrrolyl, pyrazolyl, pyrimidyl, quinolinyl, isoquinolinyl, benzofuryl, benzofuranyl, benzothiophenyl, benzothiopyranyl, benzimidazolyl, benzoxazolyl, benzooxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthyridinyl, pteridinyl, carbazolyl, azepinyl, diazepinyl, acridinyl and the like, including partially hydrogenated derivatives thereof.

The terms "halo," "halogen," and "halide" are used interchangeably herein and refer to a substituent fluoro, chloro, bromo, or iodo.

"Haloalkyl" means alkyl as defined herein in which one or more hydrogen has been replaced with same or different halogen. Exemplary haloalkyls include -CH₂Cl, -CH₂CF₃, -CH₂CCl₃, perfluoroalkyl (e.g., -CF₃), and the like.

"Heterocyclyl" means a monovalent saturated moiety, consisting of one to three rings, incorporating one, two, or three or four heteroatoms (chosen from nitrogen, oxygen or sulfur). The heterocyclyl ring may be optionally substituted as defined herein. Examples of heterocyclyl moieties include, but are not limited to, optionally substituted piperidinyl, piperazinyl, homopiperazinyl, azepinyl, pyrrolidinyl, pyrazolidinyl, imidazolinyl, imidazolidinyl, pyridinyl, pyridazinyl, pyrimidinyl, oxazolidinyl, isoxazolidinyl, morpholinyl, thiazolidinyl, isothiazolidinyl, quinuclidinyl, quinolinyl, isoquinolinyl, benzimidazolyl, thiadiazolylidinyl, benzothiazolidinyl, benzoazolylidinyl, dihydrofuryl, tetrahydrofuryl, dihydropyranyl, tetrahydropyranyl, thiamorpholinyl, thiamorpholinylsulfoxide, thiamorpholinylsulfone, dihydroquinolinyl, dihydrisoquinolinyl, tetrahydroquinolinyl, tetrahydrisoquinolinyl, and the like.

"Optionally substituted", when used in association with "aryl", phenyl", "heteroaryl" or "heterocyclyl", means an aryl, phenyl, heteroaryl or heterocyclyl which is optionally substituted independently with one or more substituents, preferably one to four, and more preferably, one to three substituents selected from C₁₋₆ alkyl, C₁₋₆ heteroalkyl, -(CH₂)ₘCOX¹, -(CH₂)ₘSO₂X², oxo (i.e., =O), haloalkyl, C₁₋₆alkoxy, halogen, C₁₋₆ alkylthio, C₁₋₆alkylsulfonyl, -SO₂NR⁴R⁵, cyano, nitro, and -NR⁶R⁷, where m, X¹, X² , R⁴ and R⁵ are as defined herein.

"Leaving group" means the group with the meaning conventionally associated with it in synthetic organic chemistry, i.e., an atom or group displaceable under substitution reaction conditions. Examples of leaving groups include, but are not limited to, halogen, alkane- or arylenesulfonyloxy, such as methanesulfonyloxy, ethanesulfonyloxy, thiomethyl, benzenesulfonyloxy, tosyloxy, and thienyloxy, dihalophosphinoyloxy, optionally substituted benzyloxy, isopropyloxy, acyloxy, and the like.

"Optional" or "optionally" means that the subsequently described event or circumstance may but need not occur, and that the description includes instances where the event or circumstance occurs and instances in which it does not.

"Disease" and "Disease state" means any disease, condition, symptom, disorder or indication.

"Inert organic solvent" or "inert solvent" means the solvent is inert under the conditions of the reaction being described in conjunction therewith, including for example, benzene, toluene, acetonitrile, tetrahydrofuran, N,N-dimethylformamide, chloroform, methylene chloride or dichloromethane, dichloroethane, diethyl ether, ethyl acetate, acetone, methyl ethyl ketone, methanol, ethanol, propanol, isopropanol, *tert*-butanol, dioxane, pyridine, and the like. Unless specified to the contrary, the solvents used in the reactions of the present invention are inert solvents.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

"Pharmaceutically acceptable salts" of a compound means salts that are pharmaceutically acceptable, as defined herein, and that possess the desired pharmacological activity of the parent compound. Such salts include:
acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid, trimethylacetic acid, and the like; or
salts formed when an acidic proton present in the parent compound either is replaced by a metal ion, e.g., an alkali metal ion, an alkaline earth ion, or an aluminum ion; or coordinates with an organic or inorganic base. Acceptable organic bases include diethanolamine, ethanolamine, N-methylglucamine, triethanolamine, tromethamine, and the like. Acceptable inorganic bases include aluminum hydroxide, calcium hydroxide, potassium hydroxide, sodium carbonate and sodium hydroxide.

The preferred pharmaceutically acceptable salts are the salts formed from acetic acid, hydrochloric acid, sulphuric acid, methanesulfonic acid, maleic acid, phosphoric acid, tartaric acid, citric acid, sodium, potassium, calcium, zinc, and magnesium.

It should be understood that all references to pharmaceutically acceptable salts include solvent addition forms (solvates) or crystal forms (polymorphs) as defined herein, of the same acid addition salt.

"Protective group" or "protecting group" means the group which selectively blocks one reactive site in a multifunctional compound such that a chemical reaction can be carried out selectively at another unprotected reactive site in the meaning conventionally associated with it in synthetic chemistry. Certain processes of this invention rely upon the protective groups to block reactive nitrogen and/or oxygen atoms present in the reactants. For example, the terms "amino-protecting group" and "nitrogen protecting group" are used interchangeably herein and refer to those organic groups intended to protect the nitrogen atom against undesirable reactions during synthetic procedures. Exemplary nitrogen protecting groups include, but are not limited to, trifluoroacetyl, acetamido, benzyl (Bn), benzyloxycarbonyl (carbobenzyloxy, CBZ), p-methoxybenzyloxycarbonyl, p-nitrobenzyloxycarbonyl, tert-butoxycarbonyl (BOC), and the like. Skilled persons will know how to choose a group for the ease of removal and for the ability to withstand the following reactions.

"Solvates" means solvent additions forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

"Subject" means mammals and non-mammals. Mammals means any member of the mammalia class including, but not limited to, humans; non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The terms "those defined above" and "those defined herein" when referring to a variable incorporates by reference the broad definition of the variable as well as preferred, more preferred and most preferred definitions, if any.

"Treating" or "treatment" of a disease state includes:
(i)preventing the disease state, i.e. causing the clinical symptoms of the disease state not to develop in a subject that may be exposed to or predisposed to the disease state, but does not yet experience or display symptoms of the disease state.
(ii)inhibiting the disease state, i.e., arresting the development of the disease state or its clinical symptoms, or
(iii)relieving the disease state , i.e., causing temporary or permanent regression of the disease state or its clinical symptoms.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

### Nomenclature and Structures

In general, the nomenclature used in this Application is based on AUTONOM^{™} v.4.0, a Beilstein Institute computerized system for the generation of IUPAC systematic nomenclature. Chemical structures shown herein were prepared using ISIS^{®} version 2.2. Any open valency appearing on a carbon, oxygen or nitrogen atom in the structures herein indicates the presence of a hydrogen atom.

Whenever a chiral carbon is present in a chemical structure, it is intended that all stereoisomers associated with that chiral carbon are encompassed by the structure.

All patents and publications identified herein are incorporated herein by reference in their entirety.

One aspect of the present invention provides compounds of formula I: 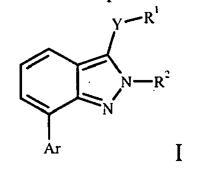 or a pharmaceutically acceptable salt thereof,
wherein:
- Y: is (CH₂)ₙ, alkenylene, CH(OR³) or C=O;
- R¹: is a five-membered heteroaryl containing at least one nitrogen ring atom, wherein said five-membered heteroaryl ring is optionally substituted with one or more substituents each of which is independently selected from the group consisting of C₁₋₆ alkyl, C₁₋ ₆ heteroalkyl, -(CH₂)ₘCOX¹, -(CH₂)ₘSO₂X², and oxo;
- X¹: is-OR³ or -NR⁴R⁵;
- X²: is C₁₋₆ alkyl or -NR⁴R⁵;
- R²: is hydrogen, C₁₋₆alkyl, C₁₋₆ haloalkyl or C₃₋₆cycloalkyl;
- Ar: is aryl or heteroaryl, each optionally substituted with one or more substituents independently selected from the group consisting of C₁₋₆ alkyl, C₁₋₆ alkoxy, C₁₋₆ alkylthio, C₁₋₆ alkylsulfonyl, -SO₂NR⁴R⁵, halogen, C₁₋₆ haloalkyl, cyano, nitro, and -NR⁶R⁷;
- each of R⁶ and R⁷: is independently selected from the group consisting of hydrogen, C₁₋₉ alkyl, and C₁₋₉alkylcarbonyl;
- each of R³, R⁴, and R⁵: is independently hydrogen or C₁₋₆ alkyl;
- m: is an integer from 0 to 4; and
n is an integer from 0 to 3.

It is to be understood that the scope of this invention encompasses not only the various isomers which may exist but also the various mixture of isomers which may be formed. Furthermore, the scope of the present invention also encompasses solvates and salts of compounds of formula I.

In certain embodiments, R¹is triazolyl, imidazolyl, tetrazolyl, pyrazolyl, or oxadiazolyl, each of which is optionally substituted.

In other embodiments, R¹ is [1,2,4]triazol-1-yl, imidazol-1-yl, tetrazol-2-yl, tetrazol-1-yl, tetrazol-5-yl, [1,2,4]triazol-3-yl, [1,2,3,]triazol-1-yl, [1,2,3,]triazol-2-yl, imidazol-2-yl, [1,2,4]triazol-4-yl, pyrazol-1-yl, [1,3,4]oxadiazol-2-yl, oxazol-5-yl, or [1,2,4]triazol-2-yl, each of which is optionally substituted.

Still in other embodiments, R¹ is 5-carbamyl-2H-[1,2,4]triazol-1-yl; 5-carbamyl-1H-imidazol-1-yl; 3-carbmethoxyl-1H-[1,2,4]triazol-1-yl; 5-carbmethoxy-1H-[1,2,4]triazol-1-yl; 5-carbethoxymethyl-2H-tetrazol-2-yl; 5-carbethoxymethyl-1H-tetrazol-1-yl; 1-methanesulfonylmethyl-1H-tetrazol-5-yl; 1-(2-hydroxylethyl)-1H-tetrazol-5-yl; 2-(2-hydroxylethyl)-2H-tetrazol-5-yl; 2-methanesulfonylmethyl-2H-tetrazol-5-yl; 5-carbmethoxy-1H-imidazol-1-yl; 5-carbethoxy-1H-imidazol-1-yl; 2H-[1,2,4]triazol-3-yl; 1H-[1,2,3]triazol-1-yl; 2H-[1,2,3]triazol-2-yl; 1H-tetrazol-5-yl; 2-methoxymethyl-2H-[1,2,4]triazol-3-yl; 1-methyl-1H-tetrazol-5-yl; 1H-imidazol-2-yl; 1H-imidazol-1-yl; 4H-[1,2,4]triazol-4-yl; 1-(2-hydroxyethyl)-1H-imidazol-2-yl; 3H-imidazol-4-yl; 1H-[1,2,4]triazol-3-yl; 1-methyl-1H-tetrazol-5-yl; 2-methyl-2H-tetrazol-5-yl; 2H-[1,2,4]triazol-3-yl; 1-(N,N-dimethyl sulfonamide)-1H-imidazol-2-yl; 1H-pyrazol-1-yl; [1,3,4]-oxadiazol-2-yl; 1H-[1,2,4]triazol-1-yl; 1H-tetrazol-1-yl; 1H-trazol-5-yl; 2H-tetrazol-2-yl; 3-methyl-[1,2,4]oxadiazol-5-yl; oxazol-5-yl; 5-carbamylmethyl-1H-tetrazol-1-yl; 3-carbamyl-1H-[1,2,4]triazol-1-yl; 3-hydroxymethyl-2H-[1,2,4]triazol-2-yl; 3-carbethoxymethyl-1H-[1,2,4]triazol-1-yl; 3-carbethoxymethyl-2H-[1,2,4]triazol-2-yl; or 5-(2-hydroxyethyl)-1H-tetrazol-1-yl.

Yet in another embodiment, Y is (CH₂)ₙ. Within this embodiment, preferably n is 0, 1 or 2.

Still in some embodiments, Y is C=O.

In other embodiments, Y is CH(OR³). Within these embodiments, R³ is preferably H, methyl, or ethyl.

In certain embodiments, Y is alkenylene. Within these embodiments, Y is preferably ethenylene.

Yet in another embodiment, R² is C₁₋₆ alkyl. Preferably, R² is methyl.

In other embodiments, Ar is disubstituted aryl or disubstituted heteroaryl. Within these embodiments, Ar is preferably disubstituted aryl, more preferably disubstituted phenyl, and still more preferably dihalide substituted phenyl, where each halide is independently selected. One particular example of Ar within compounds of formula I is 2,4-dichlorophenyl.

Yet in other embodiments, Ar is 2,4-dichlorophenyl and R² is methyl.

In certain embodiments of formula I where R² is methyl and Ar is disubstituted phenyl, the subject compounds may be represented by formula II: 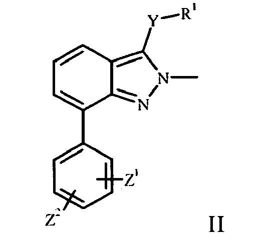 wherein
- each of Z¹ and Z²: is independently a substituent selected from C₁₋₆ alkyl, C₁₋₆ alkoxy, C₁₋₆ alkylthio, C₁₋₆ alkylsulfonyl, -SO₂NR⁴R⁵, halogen, C₁₋₆ haloalkyl, cyano, nitro, and -NR⁶R⁷; and
- Y, R¹, R⁶, R⁵, R⁶, and R⁷: are as defined herein.

In certain embodiments of formula II, R¹ is triazolyl, imidazolyl, tetrazolyl, pyrazolyl or oxadiazolyl, each of which is optionally substituted. Within these embodiments, preferably R¹ is [1,2,4]triazol-1-yl, imidazol-1-yl, tetrazol-2-yl, tetrazol-1-yl, oxazolidin-1-yl, tetrazol-5-yl, [1,2,4]triazol-3-yl, [1,2,3,]triazol-1-yl, [1,2,3,]triazol-2-yl, imidazol-2-yl, [1,2,4]triazol-4-yl, imidazol-4-yl, pyrazol-1-yl, [1,3,4]oxadiazol-2-yl, pyrrolidin-1-yl, [1,2,4]oxadiazol-5-yl, oxazol-5-yl, or [1,2,4]triazol-2-yl, each of which is optionally substituted. More preferably, R¹ is 5-carbamyl-2H-[1,2,4]triazol-1-yl; 5-carbamyl-1H-imidazol-1-yl; 3-carbmethoxyl-1H-[1,2,4]triazol-1-yl; 5-carbmethoxy-1H-[1,2,4]triazol-1-yl; 5-carbethoaymethyl-2H-tetrazol-2-yl; 5-carbethoxymethyl-1H-tetrazol-1-yl; 1-methanesulfonylmethyl-1H-tetrazol-5-yl; 1-(2-hydroxylethyl)-1H-tetrazol-5-yl; 2-(2-hydroxylethyl)-2H-tetrazol-5-yl; 2-methanesulfonylmethyl-2H-tetrazol-5-yl; 5-carbmethoxy-1H-imidazol-1-yl; 5-carbethoxy-1H-imidazol-1-yl; 2H-[1,2,4]triazol-3-yl; 1H-[1,2,3]triazol-1-yl; 2H-[1,2,3]triazol-2-yl; 1H-tetrazol-5-yl; 2-methoxymethyl-2H-[1,2,4]triazol-3-yl; 1-methyl-1H-tetrazol-5-yl; 1H-imidazol-2-yl; 1H-imidazol-1-yl; 4H-[1,2,4]triazol-4-yl; 1-(2-hydroxyethyl)-1H-imidazol-2-yl; 3H-imidazol-4-yl; 1H-[1,2,4]triazol-3-yl; 1-methyl-1H-tetrazol-5-yl; 2-methyl-2H-tetrazol-5-yl; 2H-[1,2,4]triazol-3-yl; 1-(N,N-dimethyl sulfonamide)-1H-imidazol-2-yl; 1H-pyrazol-1-yl; [1,3,4]-oxadiazol-2-yl; 1H-[1,2,4]triazol-1-yl; 1H-tetrazol-1-yl; 1H-tetrazol-5yl; 2H-tetrazol-2-yl; 3-methyl- [1,2,4]oxadiazol-5-yl; oxazol-5-yl; 5-carbamylmethyl-1H-tetrazol-1-yl; 3-carbamyl-1H-[1,2,4]triazol-1-yl; 3-hydroxymethyl-2H-[1,2,4]triazol-2-yl; 3-carbethoxymethyl-1H-[1,2,4]triazol-1-yl; 3-carbethoxymethyl-2H-[1,2,4]triazol-2-yl; or 5-2-hydroxyethyl)-1H-tetrazol-1-yl.

Yet in other embodiments of formula II, Y is (CH₂)ₙ. Within these embodiments, preferably n is 0, 1 or 2.

Still in some embodiments of formula II, Y is C=O.

In other embodiments of formula II, Y is CH(OR³). Within these embodiments, R³ is preferably H, methyl, or ethyl.

In certain embodiments of formula II, Y is alkenylene. Within these embodiments, Y is preferably ethenylene.

In other embodiments of formula II, each of Z¹ and Z² is independently halide. Within these embodiments, preferably Z¹ and Z² are chloride.

In certain embodiments of formula I where R² is methyl and Ar is disubstituted phenyl, the subject compounds may be represented by formula III: 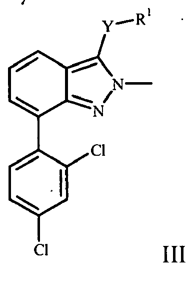 wherein Y and R¹ are as defined herein.

In certain embodiments of formula III, R¹ is triazolyl, imidazolyl, tetrazolyl, pyrazolyl, or oxadiazolyl, each of which is optionally substituted. Within these embodiments, preferably R¹ is [1,2,4]triazol-1-yl, imidazol-1-yl, tetrazol-2-yl, tetrazol-1-yl, oxazolidin-1-yl, tetrazol-5-yl, [1,2,4[triazol-3-yl, [1,2,3,]triazol-1-yl, [1,2,3,]triazol-2-yl, imidazol-2-yl, [1,2,4]triazol-4-yl, imidazol-4-yl, pyrazol-1-yl, [1,3,4]oxadiazol-2-yl, pyrrolidin-1-yl, [1,2,4]oxadiazol-5-yl, oxazol-5-yl, or [1,2,4]triazol-2-yl, each of which is optionally substituted. More preferably, R¹ is 5-carbamyl-2H-[1,2,4]triazol-1-yl; 5-carbamyl-1H-imidazol-1-yl; 3-carbmethoxyl-1H-[1,2,4]triazol-1-yl; 5-carbmethoxy-1H-[1,2,4]triazol-1-yl; 5-carbethoxymethyl-2H-tetrazol-2-yl; 5-carbethoxymethyl-1H-tetrazol-1-yl; 1-methanesulfonylmethyl-1H-tetrazol-5-yl; 1-(2-hydroxylethyl)-1H-tetrazol-5-yl; 2-(2-hydroxylethyl)-2H-tetrazol-5-yl; 2-methanesulfonylmethyl-2H-tetrazol-5-yl; 5-carbmethoxy-1H-imidazol-1-yl; 5-carbethoxy-1H-imidazol-1-yl; 2H-[1,2,4]triazol-3-yl; 1H-[1,2,3]triazol-1-yl; 2H-[1,2,3]triazol-2-yl; 1H-tetrazol-5-yl; 2-methoxymethyl-2H-[1,2,4]triazol-3-yl; 1-methyl-1H-tetrazol-5-yl; 1H-imidazol-2-yl; 1H-imidazol-1-yl; 4H-[1,2,4]triazol-4-yl; 1-(2-hydroxyethyl)-1H-imidazol-2-yl; 3H-imidazol-4-yl; 1H-[1,2,4]triazol-3-yl; 1-methyl-1H-tetrazol-5-yl; 2-methyl-2H-tetrazol-5-yl; 2H-[1,2,4]triazol-3-yl; 1-(N,N-dimethyl sulfonamide)-1H-imidazol-2-yl; 1H-pyrazol-1-yl; [1,3,4]-oxadiazol-2-yl; 1H-[1,2,4]triazol-1-yl; 1H-tetrazol-1-yl; 1H-tetrazol-5-yl; 2H-tetrazol-2-yl; 3-methyl-[1,2,4]oxadiazol-5-yl; oxazol-5-yl; 5-carbamylmethyl-1H-tetrazol-1-yl; 3-carbamyl-1H-[1,2,4]triazol-1-yl; 3-hydroxymethyl-2H-[1,2,4]triazol-2-yl; 3-carbethoxymethyl-1H-[1,2,4]triazol-1-yl; 3-carbethoxymethyl-2H-[1,2,4]triazol-2-yl; or 5-(2-hydroxyethyl)-1H-tetrazol-1-yl.

Yet in other embodiments of formula III, Y is (CH₂)ₙ. Within these embodiments, preferably n is 0, 1 or 2.

Still in some embodiments of formula III, Y is C=O.

In other embodiments of formula III, Y is CH(OR³). Within these embodiments, R³ is preferably H, methyl, or ethyl.

In certain embodiments of formula III, Y is alkenylene. Within these embodiments, Y is preferably ethenylene.

Representative compounds of the present invention, where Ar is 2,4-dichlorophenyl, are shown in TABLE 1.

| TABLE 1 | | | | | |
|---|---|---|---|---|---|
| | Structure | Method MP (°C) | MH⁺Calcd | MH⁺ Obs | Name |
| I-1 | 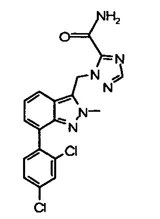 | B | 401 | 401 | 2-[7-(2,4-Dichloro-phenyl)-2-methyl-2H-indazol-3-ylmethyl]-2H- [1,2,4] triazole-3-carboxylic acid amide |
| I-2 | 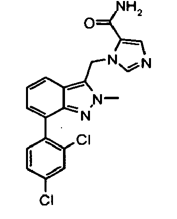 | B | 400 | 400 | 3-[7-(2,4-Dichloro-phenyl)-2-methyl-2H-indazol-3-ylmethyl]-3H-imidazole-4-carboxylic acid amide |
| I-3 | 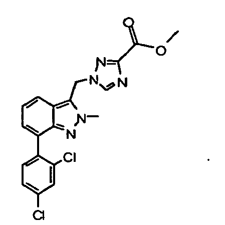 | B | 416 | 416 | 1-[7-(2,4-Dichloro-phenyl)-2-methyl-2H-indazol-3-ylmethyl] -1 H- [1,2,4]triazole-3-carboxylic acid methyl ester |
| I-4 | 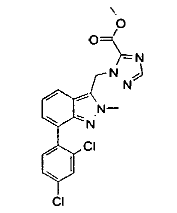 | B | 416 | 416 | 2-[7-(2,4-Dichloro-phenyl)-2-methyl-2H-indazol-3-ylmethyl]-2H- [1,2,4]triazole-3-carboxylic acid methyl ester |
| I-6 | 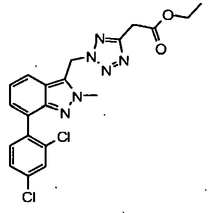 | B | 445 | 445 | {2-[7-(2,4-Dichloro-phenyl)-2-methyl-2H-indazol-3-ylmethyl]-2H-tetrazol-5-yl}-acetic acid ethyl ester |
| I-7 | 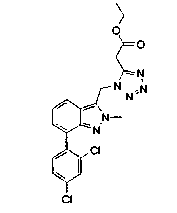 | B | 445 | 445 | {1-[7-(2,4-Dichloro-phenyl)-2-methyl-2H-indazol-3-ylmethyl]-1H-tetrazol-5-yl}-acetic acid ethyl ester |
| I-9 | 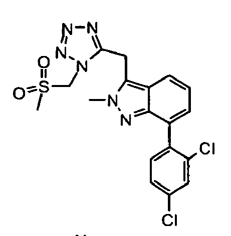 | D | 451 | 451 | 7-(2,4-Dichloro-phenyl)-3-(1-methanesulfonylmethyl-1H-tetrazol-5-ylmethyl)-2-methyl-2H-indazole |
| I-10 | 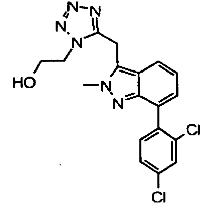 | D 66.1-87.2 | 403 | 403 | 2-{5-[7-(2,4-Dichloro-phenyl)-2-methyl-2H-indazol-3-ylmethyl]-tetrazol-1-yl}-ethanol |
| I-11 | 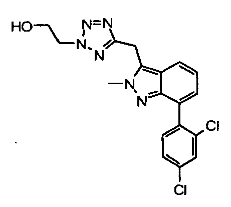 | D 147.7-151.1 | 403 | 403 | 2-{5-[7-(2,4-Dichloro-phenyl)-2-methyl-2H-indazol-3-ylmethyl]-tetrazol-2-yl}-ethanol |
| I-12 | 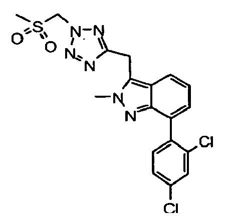 | D 156.6-157.8 | 452 | 452 | 7-(2,4-Dichloro-phenyl)-3--(2-methanesulfonylmethyl-2H-tetrazol-5-ylmethyl)-2-methyl-2H-indazole |
| I-13 | 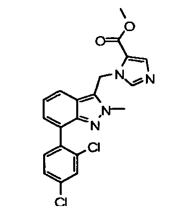 | B | 415 | 415 | 3-[7-(2,4-Dichloro-phenyl)-2-methyl-2H-indazol-3-ylmethyl]-3H-imidazole-4-carboxylic acid methyl ester |
| I-14 | 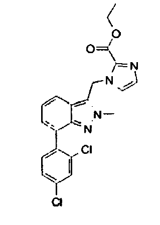 | B | 429 | 429 | 1-[7-(2,4-Dichloro-phenyl)-2-methyl-2H-indazol-3-ylmethyl] -1H-imidazole-2-carboxylic acid ethyl ester |
| I-15 | 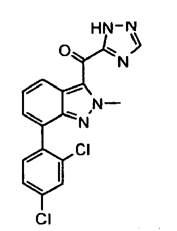 | G 283.9-284.9 | 372 | 372 | [7-(2,4-Dichloro-phenyl)-2-methyl-2H-indazol-3-yl]-(2H-[ 1,2,4]triazol-3-yl)-methanone |
| I-17 | 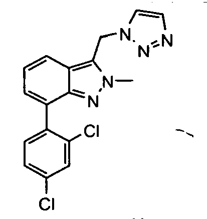 | A | 358 | 358 | 7-(2,4-Dichloro-phenyl)-2-methyl-3-[1,2,3 ]triazol-1-ylmethyl-2H-indazole |
| I-18 | 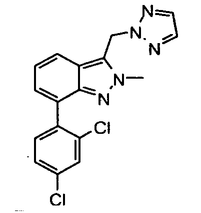 | A | 358 | 358 | 7-(2,4-Dichloro-phenyl)-2-methyl-3-[1,2,3]triazol-2-ylmethyl-2H-indazole |
| I-18 | 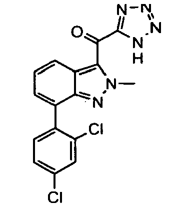 | G 209.8-210.3 | 373 | 373 | [7-(2,4-Dichloro-phenyl)-2-methyl-2H-indazol-3-yl]-(1H-tetrazol-5-yl)-methanone |
| I-19 | 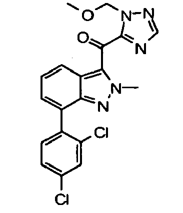 | G | 416 | 416 | [7-(2,4-Dichloro-phenyl)-2-methyl-2H-indazol-3-yl]-(2-methoxymethyl-2H-[1,2,4]triazol-3-yl)-methanone |
| I-20 | 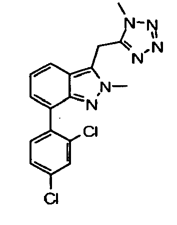 | D 183.9-185.4 | 373 | 373 | 7-(2,4-Dichloro-phenyl)-2-methyl-3-(1-methyl-1H-tetrazol-5-ylmethyl)-2H-indazole |
| I-21 | 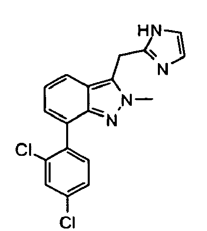 | E | 357 | 357 | 7-(2,4-Dichloro-phenyl)-3-(1H-imidazol-2-ylmethyl)-2-methyl-2H-indazole |
| I-22 | 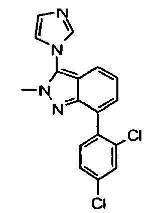 | K 63.2-89.4 | 343 | 343 | 7-(2,4-Dichloro-phenyl)-3-imidazol)-1-yl-2-methyl-2H-indazole |
| I-23 | 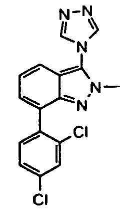 | L 117.9-121.3 | 344 | 344 | 7-(2,4-Dichloro-phenyl)-2-methyl-3-[1,2,4]triazol-4-yl-2H-indazole |
| I-24 | 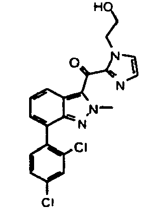 | E | 415 | 415 | [7-(2,4-Dichloro-phenyl)-2-methyl-2H-indazol-3-yl]- [1-(2-hydroxy-ethyl)-1H-imidazol-2-yl]-methanone |
| I-25 | 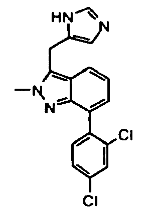 | M | 357 | 357 | 7-(2,4-Dichloro-phenyl)-3-(3H-imidazol-4-ylmethyl)-2-methyl-2H-indazole |
| I-26 | 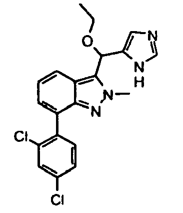 | F | 401 | 401 | 7-(2,4-Dichloro-phenyl)-3-[ethoxy-(3H-imidazol-4-yl)-methyl]-2-methyl-2H-indazole |
| I-27 | 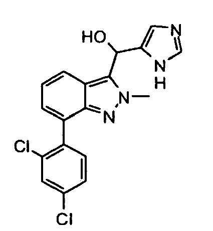 | F | 373 | 373 | [7-(2,4-Dichloro-phenyl)-2-methyl-2H-indazol-3-yl]-(3H-imidazol-4-yl)-methanol |
| I-28 | 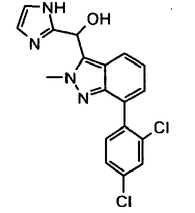 | E | 373 | 373 | [7-(2,4-Dichloro-phenyl)-2-methyl-2H-indazol-3-yl]-(1H-imidazol-2-yl)-methanol |
| I-29 | 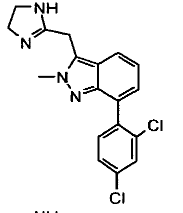 | H | 359 | 359 | 7-(2,4-Dichloro-phenyl)-3-(4,5-dihydro-1 H-imidazol-2-ylmethyl)-2-methyl-2H-indazole |
| I-30 | 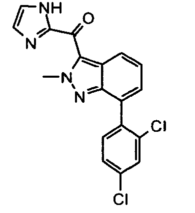 | E | 371 | 371 | [7-(2,4-Dichloro-phenyl)-2-methyl-2H-indazol-3-yl]-(1H-imidazol-2-yl)-methanone |
| I-31 | 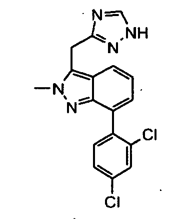 | O 206.3-207 | 358 | 358 | 7-(2,4-Dichloro-phenyl)-2-methyl-3-(1H- [1,2,4]triazol-3-ylmethyl)-2H-indazole |
| I-32 | 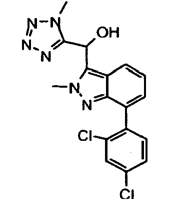 | D | 389 | 389 | [7-(2,4-Dichloro-phenyl)-2-methyl-2H-indazol-3-yl]-(1-methyl-1H-tetrazol-5-yl)-methanol |
| I-33 | 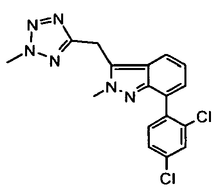 | D 158.8-160.5 | 373 | 373 | 7-(2,4-Dichloro-phenyl)-2-methyl-3-(2-methyl-2H-tetrazol-5-ylmethyl)-2H-indazole |
| I-34 | 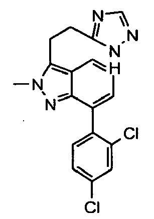 | O 92-115.5 | 372 | 372 | 7-(2,4-Dichloro-phenyl)-2-methyl-3-[2-(2H-[1,2,4]triazol-3-yl)-ethyl]-2H-indazole |
| I-35 | 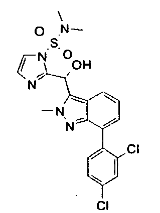 | E | 480 | 480 | 2-{[7-(2,4-Dichloro-phenyl)-2-methyl-2H-indazol-3-yl] -hydroxy-methyl{-imidazole-1-sulfonic acid dimethylamide |
| I-36 | 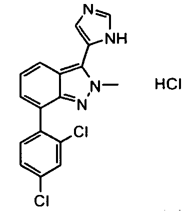 | I | 343 | 343 | 7-(2,4-Dichloro-phenyl)-3-(3H-imidazol-4-yl)-2-methyl-2H-indazole hydrochloride |
| I-37 | 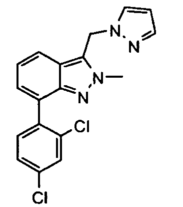 | A | 357 | 357 | 7-(2,4-Dichloro-phenyl)-2-methyl-3-pyrazol-1-ylmethyl-2H-indazole |
| I-38 | 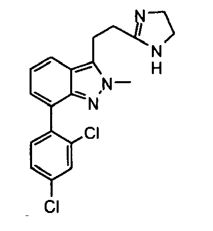 | H 60.5-65.1 | 373 | 373 | 7-(2,4-Dichloro-phenyl)-3-[2-(4,5-dihydro-1H-imidazol-2-yl)-ethyl]-2-methyl-2H-indazole |
| I-39 | 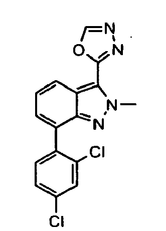 | P 199-201.2 | 345 | 345 | 7-(2,4-Dichloro-phenyl)-2-methyl-3-[1,3,4]oxadiazol-2-yl-2H-indazole |
| I-40 | 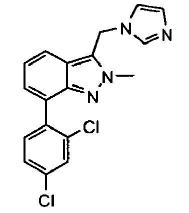 | A | 357 | 357 | 7-(2,4-Dichloro-phenyl)-3-imidazol-1-ylmethyl-2-methyl-2H-indazole |
| I-42 | 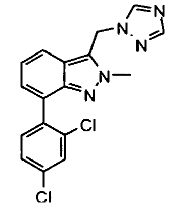 | A | 358 | 358 | 7-(2,4-Dichloro-phenyl)-2-methyl-3-[1,2,4]triazol-1-ylmethyl-2H-indazole |
| I-43 | 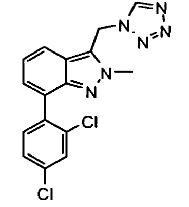 | A | 359 | 359 | 7-(2,4-Dichloro-phenyl)-2-methyl-3-tetrazol-1-ylmethyl-2H-indazole |
| I-45 | 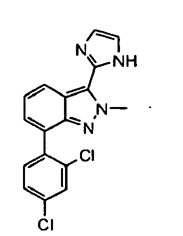 | J | 343 | 343 | 7-(2,4-Dichloro-phenyl)-3-(1H-imidazol-2-yl)-2-methyl-2H-indazole |
| I-46 | 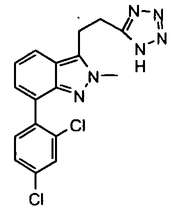 | C 209.4-210.3 | 373 | 373 | 7-(2,4-Dichloro-phenyl)-2-methyl-3-[2-(1H-tetrazol-5-yl)-ethyl]-2H-indazole |
| I-47 | 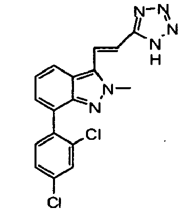 | C 256.8-258.1 | 371 | 371 | 7-(2,4-Dichloro-phenyl)-2-methyl-3-[(E)-2-(1H-tetrazol-5-yl)-vinyl]-2H-indazole |
| I-49 | 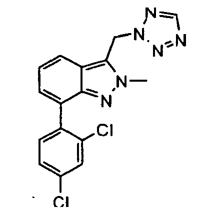 | A | 359 | 359 | 7-(2,4-Dichloro-phenyl)-2-methyl-3-tetrazol-2-ylinethyl-2H-indazole |
| I-50 | 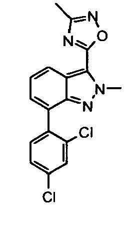 | R 178-178.5 | 359 | 359 | 7-(2,4-Dichloro-phenyl)-2-methyl-3-(3-methyl-[1,2,4]oxadiazol-5-yl)-2H-indazole |
| I-51 | 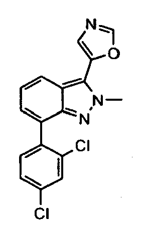 | I 167.5-169.5 | 344 | 344 | 7-(2,4-Dichloro-phenyl)-2-methyl-3-oxazol-5-yl-2H-indazole |
| I-52 | 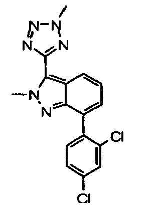 | C | 359 | 359 | 7-(2,4-Dichloro-phenyl)-2-methyl-3-(2-methyl-2H-tetrazol-5-yl)-2H-indazole |
| I-53 | 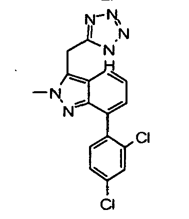 | D 270.1-275.5 | 359 | 359 | 7-(2,4-Dichloro-phenyl)-2-methyl-3-(1H-tetrazol-5-ylmethyl)-2H-indazole |
| I-54 | 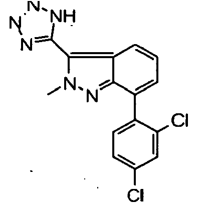 | C | 345 | 345 | 7-(2,4-Dichloro-phenyl)-2-methyl-3-(1H-tetrazol-5-yl)-2H-indazole |
| I-55 | 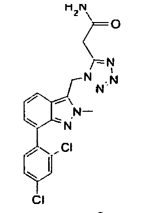 | B | 416 | 416 | 2-{1-[7-(2,4-Dichloro-phenyl)-2-methyl-2H-indazol-3-ylmethyl]-1H-tetrazol-5-yl}-acetamide |
| I-56 | 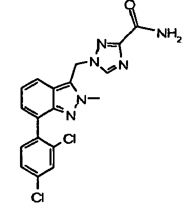 | B | 401 | 401 | 1-[7-(2,4-Dichloro-phenyl)-2-methyl-2H-indazol-3-ylmethyl]-1H- [1,2,4]triazole-3-carboxylic acid amide |
| I-57 | 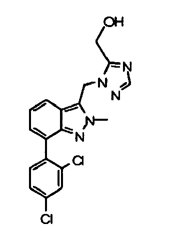 | B | 388 | 388 | {2-[7-(2,4-Dichloro-phenyl)-2-methyl-2H-indazol-3-ylmethyl]-2H-[1,2,4]triazol-3-yl}-methanol |
| I-58 | 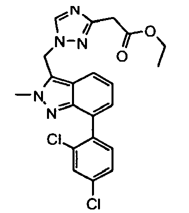 | B 80.8-84.0 | 444 | 444 | {1-[7-(2,4-Dichloro-phenyl)-2-methyl-2H-indazol-3-ylmethyl]-1H-[1,2,4]triazol-3-yl}-acetic acid ethyl ester |
| I-59 | 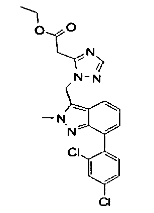 | B 167.9-168.9 | 444 | 444 | {2-[7-(2,4-Dichloro-phenyl)-2-methyl-2H-indazol-3-ylmethyl]-2H-(1,2,4]triazol-3-yl}-acetic acid ethyl ester |
| I-60 | 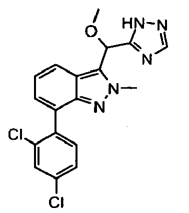 | G | 388 | 388 | 7-(2,4-Dichloro-phenyl)-3-[methoxy-(2H-[1,2,4]triazol-3-yl)-methyl]-2-methyl-2H-indazole |
| I-61 | 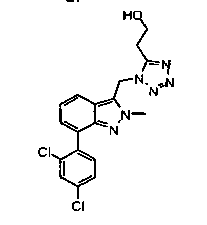 | B | 403 | 403 | 2-{1-[7-(2,4-Dichloro-phenyl)-2-methyl-2H-indazol-3-ylmethyl]-1H-tetrazol-5-yl}-ethanol |

### Synthesis

Compounds of the present invention can be made by a variety of methods depicted in the illustrative synthetic reaction schemes shown and described below. Unless specifically stated or the context requires otherwise, all the variables depicted in the illustrative synthetic schemes below are same as those defined herein.

The starting materials and reagents used in preparing these compounds generally are either available from commercial suppliers, such as Aldrich Chemical Co., or are prepared by methods known to those skilled in the art following procedures set forth in references such as Fieser and Fieser's Reagents for Organic Synthesis; Wiley & Sons: New York, 1991, Volumes 1-15; Rodd's Chemistry of Carbon Compounds, Elsevier Science Publishers, 1989, Volumes 1-5 and Supplementals; and Organic Reactions, Wiley & Sons: New York, 1991, Volumes 1-40. The following synthetic reaction schemes are merely illustrative of some methods by which the compounds of the present invention can be synthesized, and various modifications to these synthetic reaction schemes can be made and will be suggested to one skilled in the art having referred to the disclosure contained in this Application.

The starting materials and the intermediates of the synthetic reaction schemes can be isolated and purified if desired using conventional techniques, including but not limited to, filtration, distillation, crystallization, chromatography, and the like. Such materials can be characterized using conventional means, including physical constants and spectral data.

Unless specified to the contrary, the reactions described herein preferably are conducted under an inert atmosphere at atmospheric pressure at a reaction temperature range of from about -78 °C to about 150 °C, more preferably from about 0 °C to about 125 °C, and most preferably and conveniently at about room (or ambient) temperature, e.g., about 20 °C.

Scheme A below illustrates one synthetic procedure usable to prepare one of the starting materials used in preparation of compounds of the invention.

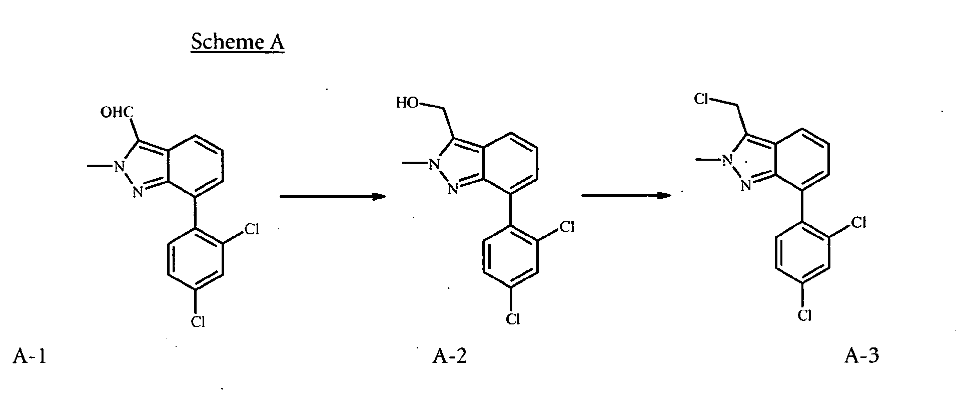

As shown in Scheme A, the aldehyde compound A-1 is reduced to an alcohol compound A-2. Reduction of an aldehyde to an alcohol is well known to one skilled in the art. Such reduction can be affected using a borohydride based reducing agents, such as sodium borohydride. Typically an alcoholic solvent, such as methanol, is used in borohydride based reduction. The alcohol compound A-2 is then converted to a chloride compound A-3, for example, by treating the alcohol with thionyl chloride. It should be appreciated by replacing the 2,4-dichlorophenyl moiety on the 7-position with other aryl or heteroaryl groups, a wide variety of different starting compounds can be prepared using the method of Scheme A.
As illustrated in Scheme B below, the chloride compound A-3 can then be used to prepare a wide variety of different R¹ groups of compounds of Formula I, II, and III.

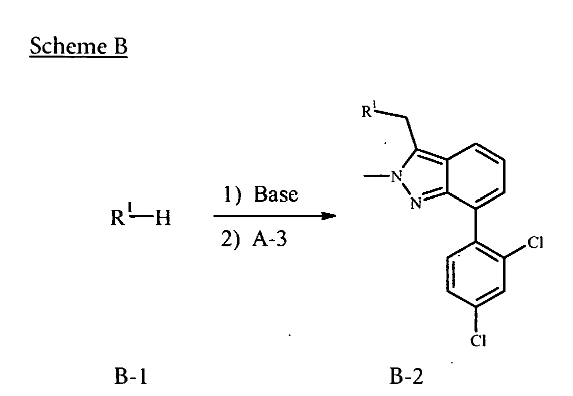

The R¹-H compound (i.e., B-1) is deprotonated using a base, such as NaH, triethylamine, DBU, etc. Treating the deprotonated R¹-H with the chloride compound A-3 (see Scheme A) then yields a variety of compounds of formula I, II, and III. This method is particularly suitable for compound of formula I, II and III, where R¹ is triazolyl, imidazolyl, pyrrolidinyl, oxazolidinyl, dihydro imidazolyl, pyrazolyl, oxadiazolyl, dihydro oxazolyl, or oxadiazolyl, and the nitrogen atom of R¹ is attached to the proton shown in Scheme B. Optionally, any functional group present in the R¹ group can be further transformed, if desired to produce other compounds of formula I, II or III.

Scheme C below illustrate one method for producing compounds of formula I, II, and III, where R¹ is a tetrazole moiety.

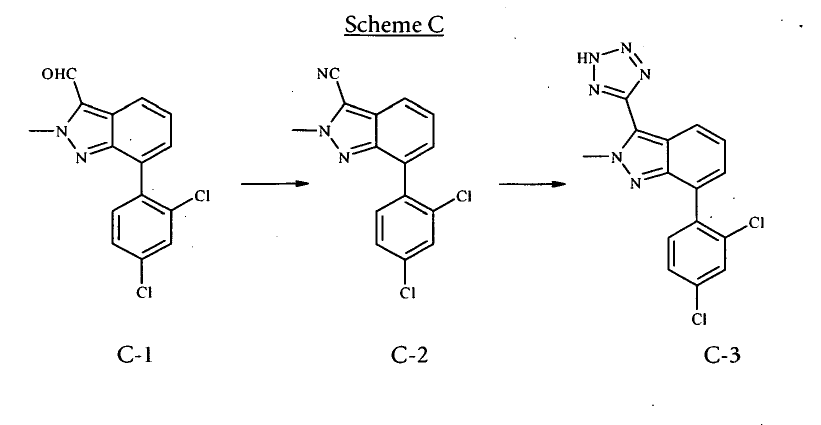

The aldehyde group (i.e., C=O moiety) of compound C-1 (same as A-1 in Scheme A above) is converted to a cyano group, for example, by treating compound C-1 with ammonia in the presence of manganese oxide and magnesium sulfate. Typically, this reaction is carried out in isopropanol-tetrahydrofuran solvent mixture at room temperature. The cyano group of compound C-2 is then converted to a tetrazol moiety by treating compound C-2 with trialkyltin azide, for example, such as Bu₃SnN₃ in toluene solvent at an elevated temperature, e.g., 100 °C. If desired, the tetrazole moiety of compound C-3 can be alkylated to provide various alkyl, substituted tetrazole moieties.

Scheme D illustrates another method for producing compounds of formula I, II, and III.

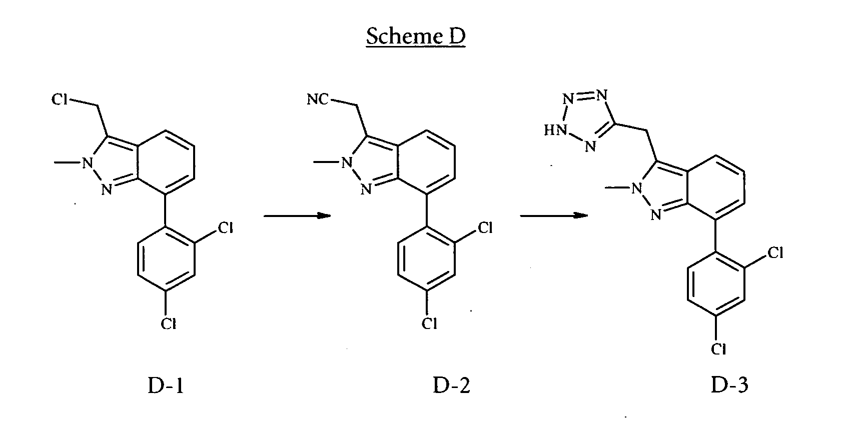

In Scheme D, compound D-1 (same as compound A-3 in Scheme A) is treated with, for example, sodium cyanide in dimethylsulfoxide to produce the cyano compound D-2. Compound D-2 is then converted to the tetrazole compound D-3 as described above in reference to Scheme C.

Scheme E below illustrates yet another method for producing compounds of the present invention.

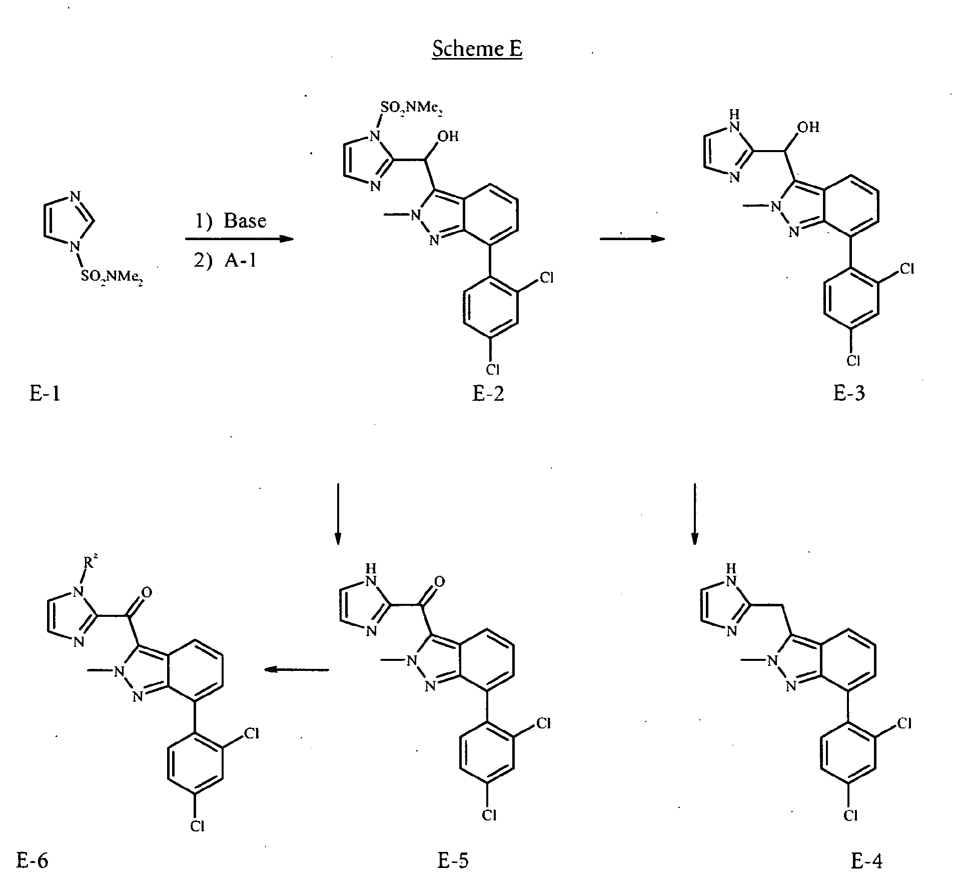

Treating nitrogen protected compound E-1 with a base, such as butyllithium, followed by compound A-1 (see Scheme A) provides an alcohol E-2. Removing the nitrogen protecting group, for example, by treating with sulfuric acid, then gives the deprotected compound E-3. The alcohol moiety of compound E-3 can be removed (i.e., reduce) by treating E-3 with trialkylsilane, such as triethylsilane, in the presence of trifluoroacetic acid. Reduction of an alcohol moiety to a saturated alkyl group is well known to one skilled in the art. Any suitable alcohol reduction reaction can be used to convert compound E-3 to E-4. Alternatively, oxidation of the alcohol moiety of compound E-2, for example with manganese oxide, and removal of the nitrogen protecting group provides the ketone compound E-5. The nitrogen atom of compound E-5 can be further derivatized using any one of the wide variety of reactions known to one skilled in the art to produce the compound E-6.

As shown in Scheme E above, using the compound E-1 provides imidazol-2-yl moiety. It should be appreciated that by using a 1,2-diprotected imidazole compound, one can produce imidazol-4-yl moiety as shown in Scheme F below.

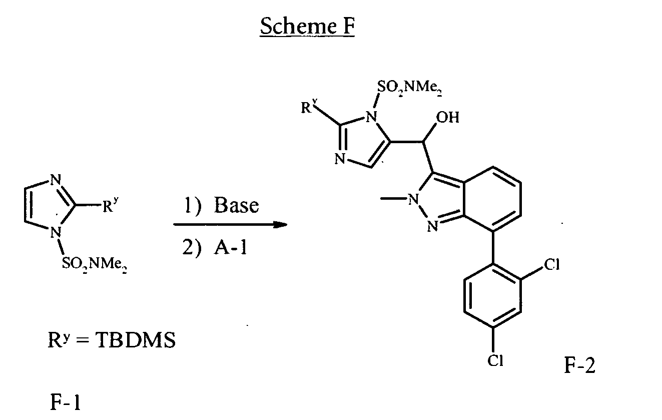

Similarly, one can introduce a wide variety of R¹ moieties by protecting appropriate position(s) of moiety R¹.

Scheme G below illustrate one method of introducing 4,5-dihydro-2H-imidazol-2-yl moiety.

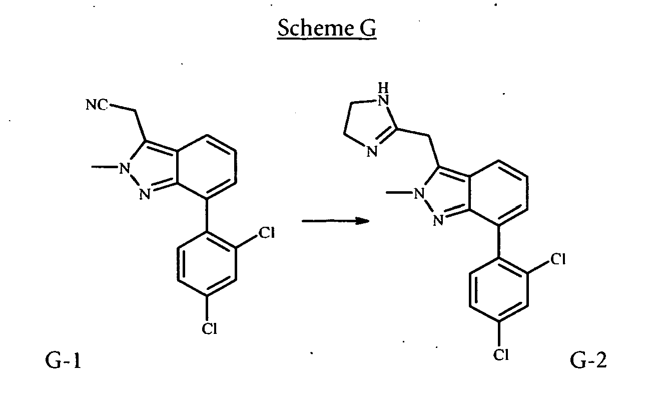

Treating compound G-1 (same as D-2 of Scheme D) with 1,2-diaminoethane in the presence of P₂S₅ provides compound G-2, which can be further modified, for example, the nitrogen atom of the dihydroimidazol moiety can be alkylated or acylated using any of the standard alkylating or acylating conditions known to one skilled in the art.

Another method for producing some of the compounds of the present invention is shown in Scheme H below.

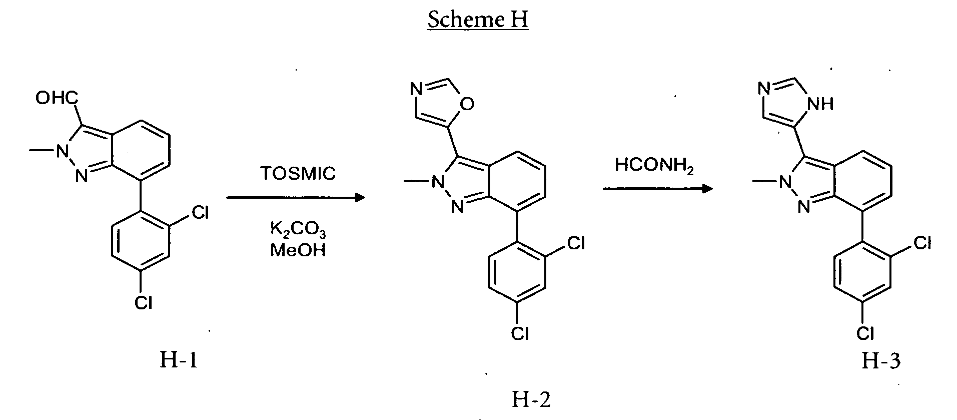

Compound H-1 (same as A-1 of Scheme A) is treated with tosylmethylisocyanide in the presence of potassium carbonate to produce the oxazolidinyl moiety from the aldehyde functional group. Treating compound H-2 with formamide produces imidazolyl substituted compound H-3.

Another method for producing some of the compounds of the present invention is illustrated in Scheme I below.

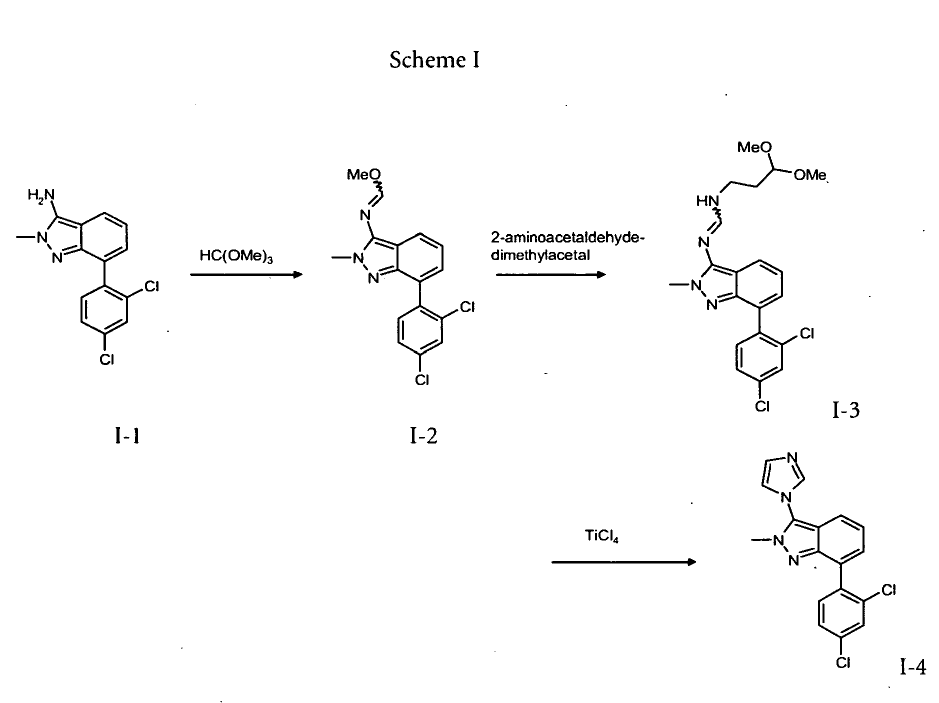

Treating compound I-1 with trimethyl orthoformate yields an imine compound I-2. Reacting the imine compound I-2 with 2-aminoacetaldehyde dimethylacetal then provides compound I-3 which is then cyclized using titanium tetrachloride to provide compound I-4.

Alternatively, treating compound I-1 with 1,2-diformylhydrazine in the presence of trimethylsilyl chloride and a base provides a triazole substituted compound 1-5, as shown below.

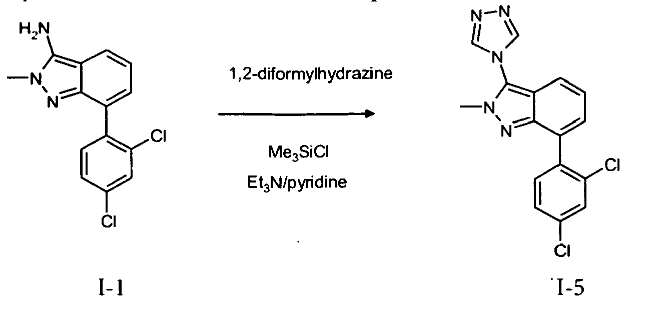

Yet another method for producing some of the compounds of the present invention is illustrated in Scheme J below.

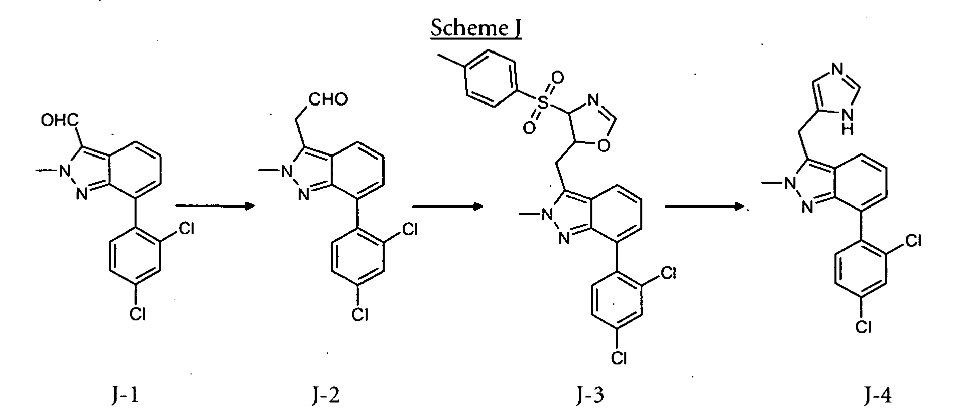

In scheme J, compound Ph₃PCH₂OCH₃ is first treated with butyllithium. The resulting anion is then treated with the aldehyde compound J-1 to produce homologated aldehyde compound J-2.Reacting the aldehyde J-2 with tosylmethylisocyanide then provides dihydrooxazolidinyl substituted compound J-3. Compound J-3 can be converted to a imidazolyl substituted compound J-4 by treatment with ammonia in isopropanol solvent.

Still another method for producing some of the compounds of the present invention is illustrated in Scheme K below.

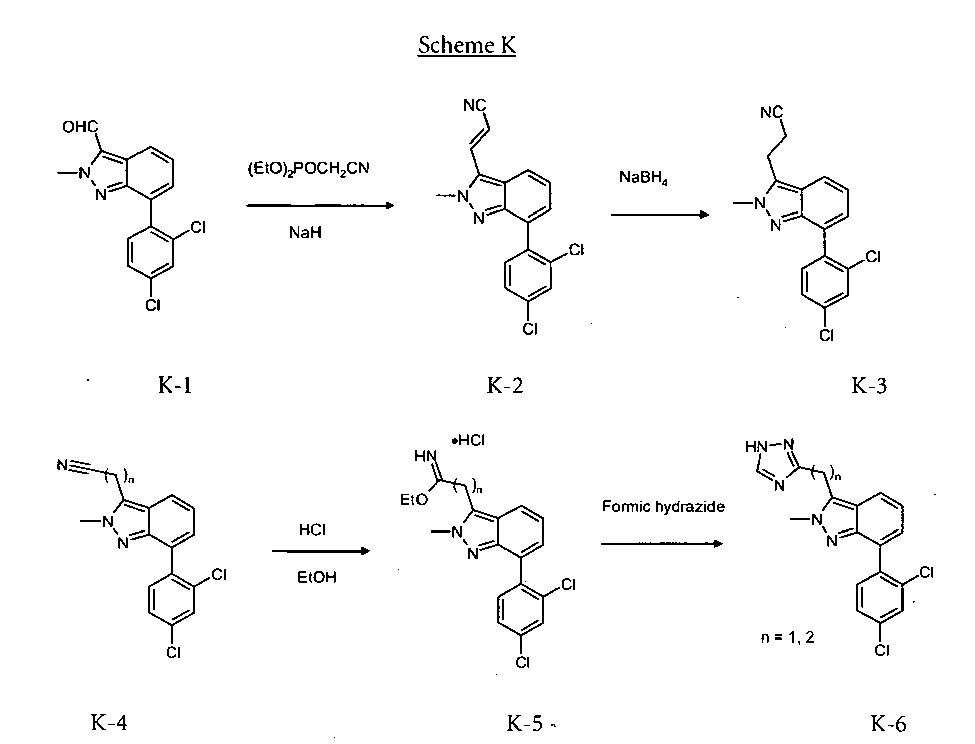

Deprotonating (EtO)₂POCH₂CN with a base, for example, sodium hydride, and reacting the resulting anion with the aldehyde compound K-1 (same as A-1 in Scheme A) produces a cyanoacrylate compound K-2. Reduction of the double bond, for example, with sodium borohydride, then provides a cyano compound K-3. Whether the cyano compound is tethered to indazole core moiety by methylene or ethylene moiety, it can be converted to a triazole moiety following the method illustrated in the second half of Scheme K. Thus, treating the cyano compound K-4 with hydrochloric acid produces an imine compound K-5. The imine moiety is then converted to a triazole moiety by treating the compound K-5 with formic hydrazide.

Some of the dihydrooxazolyl or oxadiazolyl substituted compounds of the present invention can be produced in accordance with the method illustrated in Scheme L below.

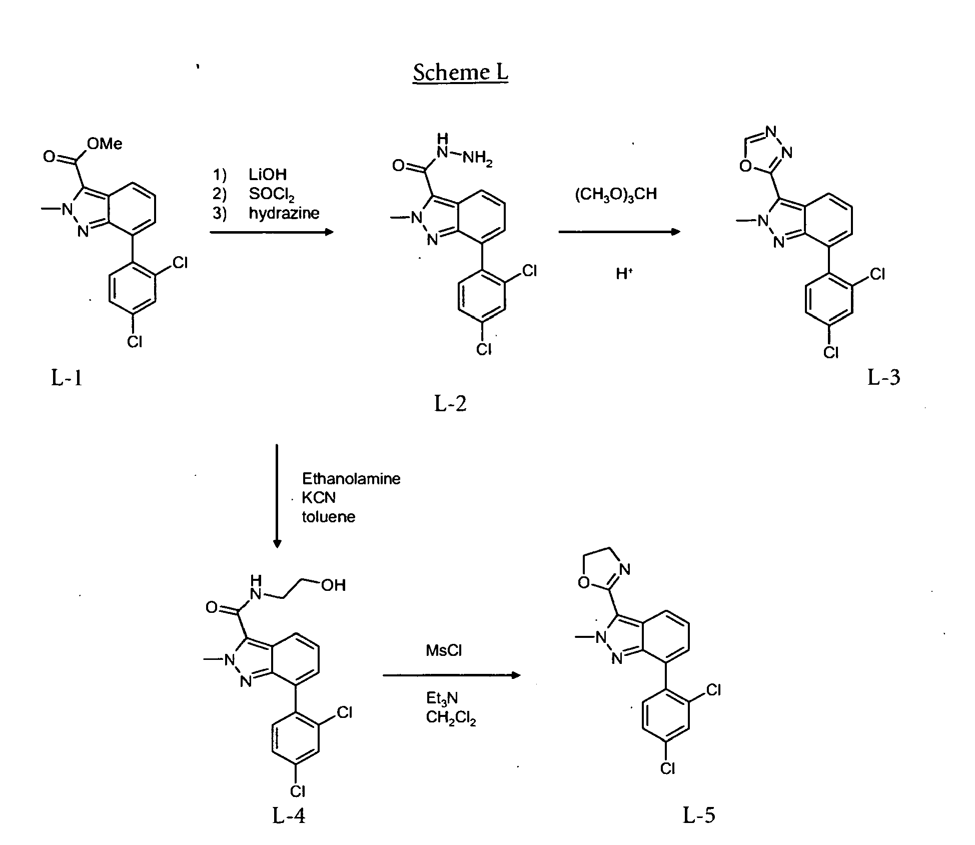

The ester compound L-1 can be converted to a hydrazide compound L-2 by converting the ester to an acyl halide and reacting the activated acyl compound with hydrazine. Reacting the hydrazide compound L-2 with trimethyl orthoformate then affords [1,3,4]-oxadiazolyl compound L-3. Alternatively, acyl transfer reaction of the compound L-I with ethanolamine affords an amide compound L-4. Intramolecular cyclization of amide L-4 then affords 4,5-dihydrooxazole compound L-5. The intramolecular cyclization reaction is typically achieved by treating the amide L-4 with mesyl chloride in the presence of a base, such as triethyl amine.

Scheme M below illustrate one method for producing [1,3,4]-oxadiazolyl substituted indazole compounds of the present invention.

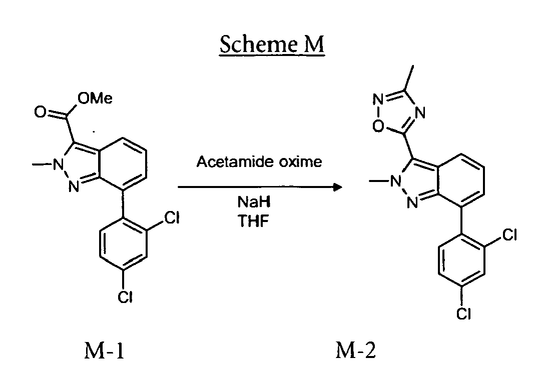

Treatment of acetamide oxime with a base, such as sodium hydride, followed by adding the ester compound M-1 produces the [1,3,4]-oxadiazolyl moiety.

Scheme N below shows one method for synthesizing 2,5-dioxopyrrolidinyl (i.e., pyrrolidine-2,5-dione) substituted compounds of formula I, II and III.

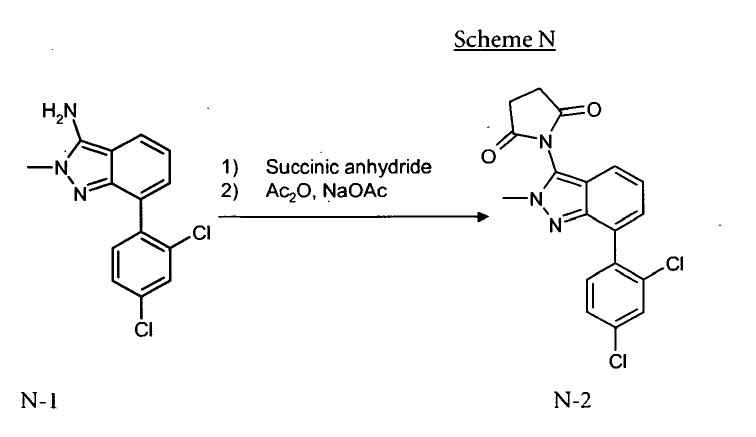

Reacting the amino moiety with succinic anhydride followed by intramolecular cyclization using acetic anhydride produces the 2,5-dioxopyroolidinyl substituted indazole N-2.

Numerous variations on the procedures of Schemes A through N are possible and will be readily apparent to those skilled in the art. For example, 2,4-dichlorophenyl moiety in the indazole ring system can be replaced by other aryl and heteroaryl moieties. While methyl and ethyl esters are often shown in the schemes above, it should be readily apparent, however, that other esters, such as propyl, isopropyl, butyl or other alkyl esters, may be used in place thereof.

Specific details for producing compounds of the invention are described in the Examples section below.

### Utility

The compounds of this invention are positive allosteric modulator of a GABA_{A} receptor which selectively modulate the α₂ subtype in the presence of the α₁ subtype, and as such are expected to be effective in the treatment of a wide range of stress-related illnesses, mood disorders such treatment and/or prevention of anxiety disorders, such as panic disorder with or without agoraphobia, agoraphobia without history of panic disorder, phobias including social phobias, obsessive-compulsive disorder, stress disorders including post-traumatic and acute stress disorder, and generalized or substance-induced anxiety disorder; depression or bipolar disorders such as single-episode or recurrent major depressive disorder, dysthymic disorder, bipolar I and bipolar II manic disorders; schizophrenia; learning and cognitive disorder such as Alzheimer's disease and attention deficit hyperactivity disorder; and sleep disorders and disorders of circadian rhythm, e.g. in subjects suffering from the effects of jet lag or shift work; convulsive or seizure disorders such as epilepsy and pain.

These and other therapeutic uses are described, for example, in Goodman & Gilman's, The Pharmacological Basis of Therapeutics, tenth edition, McGraw-Hill, New York, 2001, Chapter 19:447-483.

### Administration and Pharmaceutical Composition

The invention includes pharmaceutical compositions comprising at least one compound of the present invention, or an individual isomer, racemic or non-racemic mixture of isomers or a pharmaceutically acceptable salt or solvate thereof, together with at least one pharmaceutically acceptable carrier, and optionally other therapeutic and/or prophylactic ingredients.

In general, the compounds of the invention will be administered in a therapeutically effective amount by any of the accepted modes of administration for agents that serve similar utilities. Suitable dosage ranges are typically I-500 mg daily, preferably 1-100 mg daily, and most preferably 1-30 mg daily, depending upon numerous factors such as the severity of the disease to be treated, the age and relative health of the subject, the potency of the compound used, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this Application, to ascertain a therapeutically effective amount of the compounds of the present invention for a given disease.

Compounds of the invention may be administered as pharmaceutical formulations including those suitable for oral (including buccal and sub-lingual), rectal, nasal, topical, pulmonary, vaginal, or parenteral (including intramuscular, intraarterial, intrathecal, subcutaneous and intravenous) administration or in a form suitable for administration by inhalation or insufflation. The preferred manner of administration is generally oral using a convenient daily dosage regimen which can be adjusted according to the degree of affliction.

A compound or compounds of the invention, together with one or more conventional adjuvants, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. Formulations containing about one (1) milligram of active ingredient or, more broadly, about 0.01 to about one hundred (100) milligrams, per tablet, are accordingly suitable representative unit dosage forms.

The compounds of the invention may be formulated in a wide variety of oral administration dosage forms. The pharmaceutical compositions and dosage forms may comprise a compound or compounds of the present invention or pharmaceutically acceptable salts thereof as the active component. The pharmaceutically acceptable carriers may be either solid or liquid. Solid form preparations include powders, tablets, pills, capsules, cachets, suppositories, and dispersible granules. A solid carrier may be one or more substances which may also act as diluents, flavouring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. The powders and tablets preferably contain from about one (1) to about seventy (70) percent of the active compound. Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting wax, cocoa butter, and the like. The term "preparation" is intended to include the formulation of the active compound with encapsulating material as carrier, providing a capsule in which the active component, with or without carriers, is surrounded by a carrier, which is in association with it. Similarly, cachets and lozenges are included. Tablets, powders, capsules, pills, cachets, and lozenges may be as solid forms suitable for oral administration.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents. Aqueous suspensions can be prepared by dispersing the finely divided active component in water-with viscous material, such as natural or synthetic gums, resins, methylcellulose, sodium carboxymethylcellulose, and other well known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The compounds of the invention may be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, for example solutions in aqueous polyethylene glycol. Examples of oily or nonaqueous carriers, diluents, solvents or vehicles include propylene glycol, polyethylene glycol, vegetable oils (e.g., olive oil), and injectable organic esters (e.g., ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilization from solution for constitution before use with a suitable vehicle, e.g., sterile, pyrogen-free water.

The compounds of the invention may be formulated for topical administration to the epidermis as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatine and glycerine or sucrose and acacia; and mouthwashes comprising the active ingredient in a suitable liquid carrier.

The compounds of the invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The compounds of the invention may be formulated for vaginal administration. Pessaries, tampons, creams, gels, pastes, foams or sprays containing in addition to the active ingredient such carriers as are known in the art to be appropriate.

The subject compounds may be formulated for nasal administration. The solutions or suspensions are applied directly to the nasal cavity by conventional means, for example, with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved for example by means of a metering atomizing spray pump.

The compounds of the invention may be formulated for aerosol administration, particularly to the respiratory tract and including intranasal administration. The compound will generally have a small particle size for example of the order of five (5) microns or less. Such a particle size may be obtained by means known in the art, for example by micronization. The active ingredient is provided in a pressurized pack with a suitable propellant such as a chlorofluorocarbon (CFC), for example, dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form for example in capsules or cartridges of e.g., gelatine or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to an skin-adhesive solid support. The compound of interest can also be combined with a penetration enhancer, e.g., Azone (1-dodecylazacycloheptan-2-one). Sustained release delivery systems are inserted subcutaneously into the subdermal layer by surgery or injection. The subdermal implants encapsulate the compound in a lipid soluble membrane, e.g., silicone rubber, or a biodegradable polymer, e.g., polylactic acid.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Other suitable pharmaceutical carriers and their formulations are described in Remington: The Science and Practice of Pharmacy 1995, edited by E. W. Martin, Mack Publishing Company, 19th edition, Easton, Pennsylvania. Representative pharmaceutical formulations containing a compound of the present invention are described below.

Additional objects, advantages, and novel features of this invention will become apparent to those skilled in the art upon examination of the following examples thereof, which are not intended to be limiting.

### Examples

### Example 1 (Method A)

### Synthesis of 7-Bromo-2-methyl-2H-indazole-3-carbaldehyde.

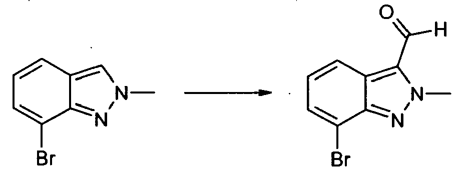

A stirred solution of 7-bromo-2-methyl-2H-indazole (8.1 g, 38 mmol) in dry THF (75 mL) was cooled to -78°C and 2 M solution of lithium diisopropyl amide (i.e., LDA) (34.5 mL, 1.8 eq) was added. The reaction was stirred 90 min at -78°C, 25 min at 0 °C, cooled back to -78°C, dimethylformamide (i.e., DMF) (9 mL) was added dropwise and the reaction was allowed to warm to room temperature overnight. The reaction was quenched with NH₄Cl (aq.), the organics extracted with ethyl acetate, dried (Na₂SO₄), filtered, and the solvent removed. The product precipitated out of ethyl acetate and the remainder was purified on a silica gel column using hexanes/ethyl acetate to give of combined total of 6.52 g (71% yield).

### Synthesis of 7-(2,4-Dichlorophenyl)-2-methyl-2H-indazole-3-carbaldehyde.

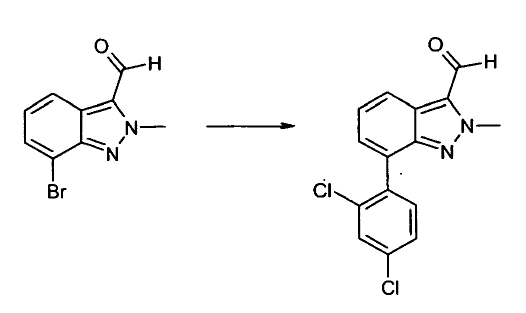

Ethylene glycol dimethyl ether (180 mL), 0.5 M Na₂CO₃ (165 mL), 7-bromo-2-methyl-2H indazole-3-carbaldehyde (6.52 g, 27 mmol), 2,4-dichlorobezeneboronic acid (6.77 g, 1.3 eq.), and tetrakis(triphenylphosphine) palladium (947 mg, 3 mol %) were combined and stirred for 2.5 h at 85° C. The reaction mixture was allowed to cool and partitioned between ethyl acetate and water/brine, extracted with ethyl acetate, dried (Na₂SO₄), filtered and the solvent removed. The product precipitated out of ethyl acetate and the remainder was purified on a silica gel column using hexanes/ethyl acetate to give a combined total of 7.87 g (95% yield).

### Synthesis of 3-Chloromethyl-7-(2,4-dichlorophenyl)-2-methyl-2H-indazole.

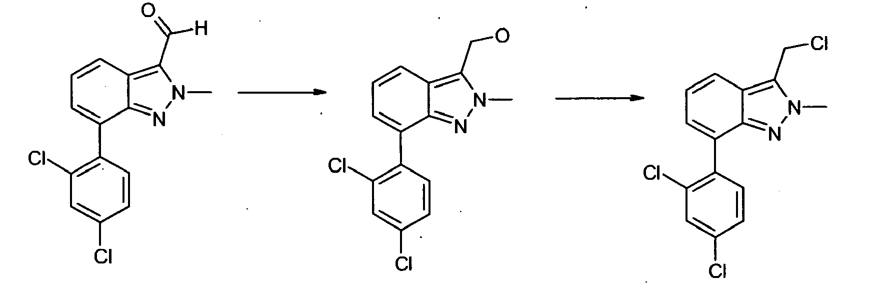

To a slurry of 7-(2,4-dichlorophenyl)-2-methyl-2H-indazole-3-carbaldehyde (1g, 3.3 mmol) was added NaBH₄ (151 mg, 1.2 eq.) in portions over a 3 min period. The solution was stirred for 1 h, partitioned between 1 M HCl and ethyl acetate, extracted with ethyl acetate, dried (Na₂SO₄), filtered and the solvent removed. The crude material was suspended in CHCl₃ (40 mL), SOCl₂ (1.2 g) added, and the reaction stirred for 2 h at 60° C. The reaction was allowed to cool, solvent was stripped off and the product purified on a silica gel column using hexanes/ethyl acetate to give 701 mg (66% yield).

### Synthesis of 7-(2,4-Dichlorophenyl)-2-inethyl-3-[1,2,4]triazol-1-ylmethyl-2H-indazole.

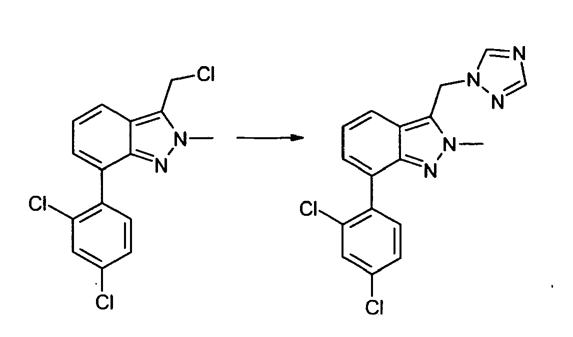

To a solution of 1,2,4-triazole (92 mg, 1.1 eq.) in dry DMF (5 mL) was added NaH (60 mg, 60% dispersion in mineral oil), stirred for 10 min, and a solution of 3-chloromethyl-7-(2,4-dichlorophenyl)-2-methyl-2H-indazole (392 mg, 1.2 mmol) in dry DMF (3 mL) was added and the reaction stirred for 1 h at 80° C. The reaction was poured into 3 M aqueous solution of NH₄Cl and extracted with ethyl acetate. The extracts were washed with water and brine, dried (Na₂SO₄), filtered, and the solvent removed. The product was purified on a silica gel column using ethyl acetate to give 361 mg (84% yield).

### Example 2 (Method B)

### Synthesis of 2-[7-(2,4-Dichlorophenyl)-2-methyl-2H-indazol-3-ylmethyl]-2H-[1,2,4]triazole-3-carboxylic acid methyl ester (I-4) and 1-[7-(2,4-dichlorophenyl)-2-methyl-2H-indazol-3-ylmethyl]-1H-[1,2,4]triazole-3-carboxylic acid methyl ester (I-3).

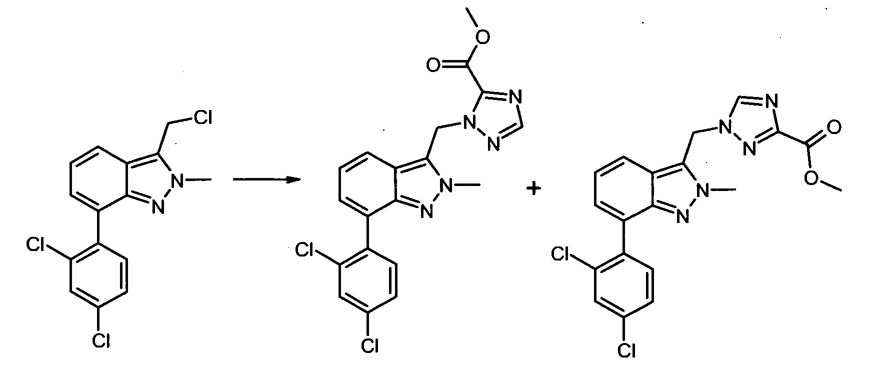

To a solution of methyl 1,2,4-triazole-3-carboxylate (54 mg, 1.15 eq.) in dry DMF (3 mL) was added NaH (19 mg, 60% dispersion in mineral oil) and the mixture stirred for 10 min. A solution of 3-chloromethyl-7-(2,4-dichlorophenyl)-2-methyl-2H-indazole (120 mg, 0.4 mmol) in dry DMF (3 mL) was added to the mixture and the reaction was stirred for 45 min at 80° C. The reaction mixture was poured into 3 M NH₄Cl and extracted with ethyl acetate. The extracts were washed with water and brine, dried (Na₂SO₄), filtered, and the solvent removed. The products were purified on a silica gel column using hexanes/ethyl acetate to give 59 mg of each of the title isomers (79% yield).

### Synthesis of 2-[7-(2,4-Dichlorophenyl)-2-methyl-2H-indazol-3-ylmethyl]-2H-[1,2,4]triazole-3-carboxylic acid amide (I-1).

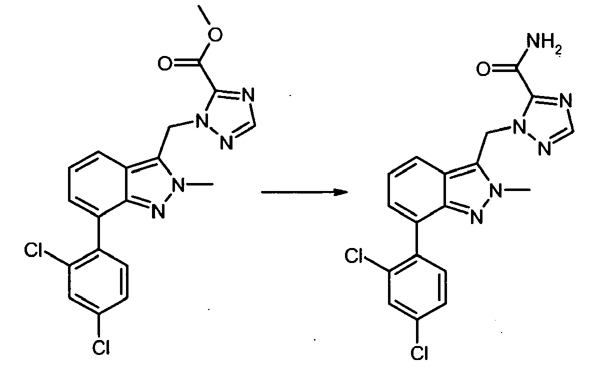

A mixture of 2- [7-(2,4-dichlorophenyl)-2-methyl-2H-indazol-3-yhnethyl]-2H-[1,2,4]triazole-3-carboxylic acid methyl ester (87 mg, 0.2 mmol), NaCN (10 mg), and 7 M NH₃ in methanol (30 mL) were combined in a sealed flask and stirred overnight at 50° C. The solvent was removed and the residue purified on a silica gel column using methylene chloride to give 71 mg of the product (85% yield).

### Synthesis of 2-[7-(2,4-Dichlorophenyl)-2-methyl-2H-indazol-3-ylmethyl]-2H-[1,2,4]triazol-3-yl-methanol, (I-57).

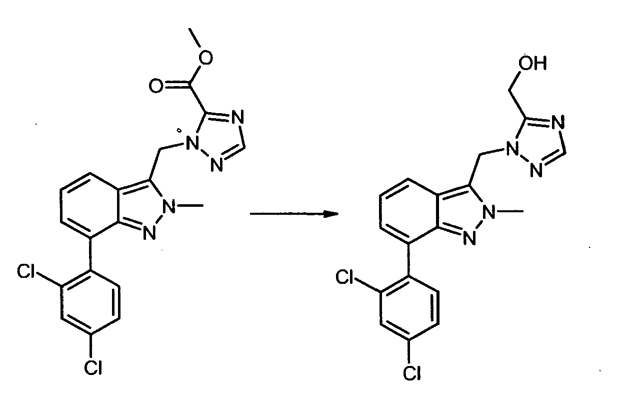

A mixture of 2-[7-(2,4-dichlorophenyl)-2-methyl-2H-indazol-3-ylmethyl]-2H-[1,2,4]triazole-3-carboxylic acid methyl ester (87 mg, 0.2 mmol), and LiBH₄ (10 mg), were stirred overnight in methanol (20 mL). The reaction was poured into water and extracted with 10% butanol in methylene chloride. The solvent was removed and the residue purified on a silica gel column using methylene chloride/methanol to give 41 mg of product (51% yield).

### Example 3 (Method C)

### Synthesis of 7-(2,4-Dichloro-phenyl)-2-methyl-2H-indazole-3-carbonitrile

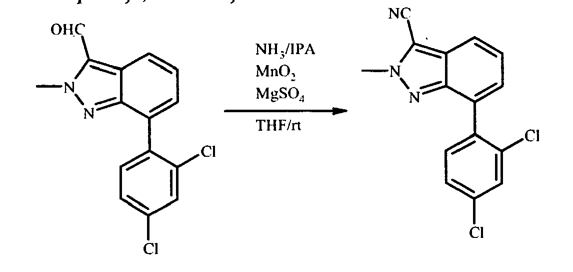

To a THF (7 mL) solution of aldehyde compound (548 mg, 1.79 mmol) was added NH₃/IPA (2 M, 3.6 mL, 7.2 mmol). MnO₂ (1.56 g, 17.94 mmol) and MgSO₄ (3.22 g, 26.7 mmol) were then added. The mixture was stirred at room temperature overnight and diluted with CH₂Cl₂. The reaction mixture was filtered through Celite and the solids were washed with CH₂Cl₂ twice. The filtrate was concentrated and the residue purified on SiO₂, eluting with 10-18% EtOAc in hexanes over 25 minutes to give a white solid product (403 mg, 74%).

### Synthesis of 7-(2,4-Dichloro-phenyl)-2-methyl-3-(2H-tetrazol-5-yl)-2H-indazole (I-54)

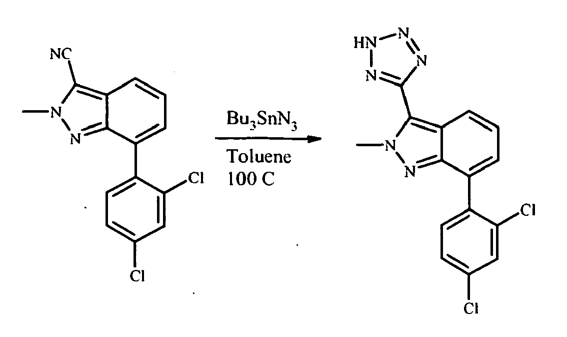

To a toluene (5 mL) solution of nitrile compound (403 mg, 1.33 mmol) was added Bu₃SnN₃ (0.55 mL, 2 mmol). The mixture was stirred at 100°C overnight and cooled to room temperature. The mixture was partitioned between water and CHCl₃. the aqueous layer was extracted with CHCl₃. The combined organic layers were dried (MgSO₄) and concentrated. The residue was purified on SiO₂, eluting with 4% MeOH in CH₂Cl₂ containing 0.5% of HOAc to give the product (250 mg, 54%).

### Synthesis of 7-(2,4-Dichloro-phenyl)-2-methyl-3-(2-methyl-2H-tetrazol-5-yl)-2H-indazole (I-52)

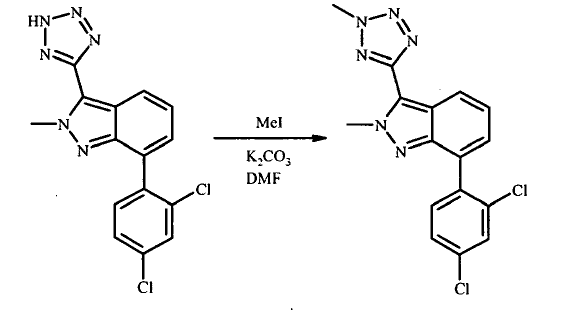

To a DMF (2 mL) solution of tetrazole (250 mg, 0.726 mmol) was added MeI (0.23 mL, 3.69 mmol) and K₂CO₃ (1.13 g, 8.18 mmol). The mixture was stirred at room temperature for 2 days and partitioned between EtOAc and water. The organic layer was dried (MgSO₄) and concentrated. The residue was purified on SiO₂, eluting with 10-18% EtOAc in hexanes over 25 minutes to give the product (116 mg, 44%).

### Example 4 (Method D)

### Synthesis of [7-(2,4-Dichloro-phenyl)-2-methyl-2H-indazol-3-yl]-acetonitrile

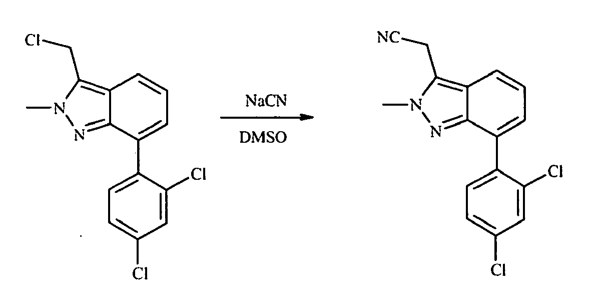

To a DMSO (50 mL) solution of chloride (2.27 g, 7.0 mmol) was added NaCN (1.2 g, 24.5 mmol). The mixture was stirred at 65°C for 1h and partitioned between EtOAc and water. The organic layer was dried (MgSO₄) and concentrated. The residue was purified on SiO₂, eluting with 35-50% EtOAc in hexanes over 30 minutes to give the product (1.22 g, 55%).

### Synthesis of 7-(2,4-Dichloro-phenyl)-2-methyl-3-(2H-tetrazol-5-ylmethyl)-2H-indazole (I-53)

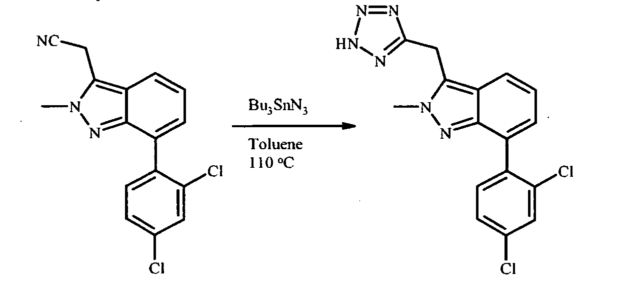

To a toluene (20 mL) solution of nitrile (1.22 g, 3.86 mmol) was added Bu₃SnN₃ (1.80 mL, 6.57 mmol). The mixture was stirred at 110 °C overnight and cooled to room temperature. The mixture was partitioned between water and CHCl₃. the aqueous layer was extracted with CHCl₃. The combined organic layers were dried (MgSO₄) and concentrated. The residue was purified on SiO₂, eluting with 10% MeOH in CH₂Cl₂ containing 0.5% of HOAc to give the product (1.25 g, 90%).

### Sytititesis of 7-(2,4-Dichloro-phenyl)-2-methyl-3-(2-methyl-2H-tetrazol-5-ylmethyl)-2H-indazole (A, I-33) and [7-(2,4-Dichloro-phenyl)-2-methyl-2H-indazol-3-yl]-(1-methyl-1H-tetrazol-5-yl)-methanol (B, I-32)

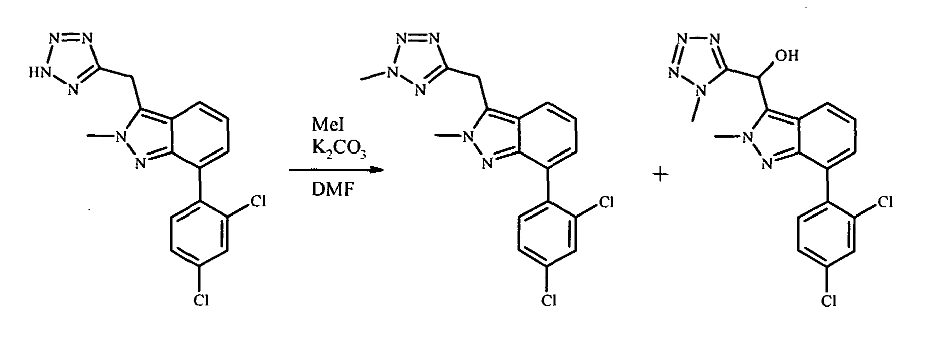

To a DMF (3 mL) solution of tetrazole (154 mg, 0.430 mmol) was added MeI (0.20 mL, 3.20 mmol) and K₂CO₃ (0.66 g, 4.77 mmol). The mixture was stirred at room temperature overnight and partitioned between EtOAc and water. The organic layer was dried (MgSO₄) and concentrated. The residue solidified. Trituration with 10% EtOAc in hexanes and filtration yielded product A (47 mg, 29%). The filtrate was purified by preparative TLC (0.5 mm thickness, 20 x 20 cm², 50% EtOAc/hexanes) to give product B (42 mg, 25%).

### Synthesis of 7-(2,4-Dichloro-phenyl)-2-methyl-3-(2-methylsulfanylmethyl-2H-tetrazol-5-ylmethyl)-2H-indazole (A) and 7-(2,4-Dichloro-phenyl)-2-methyl-3-(1-methylsulfanylmethyl-1H-tetrazol-5-ylmethyl)-2H-indazole (B)

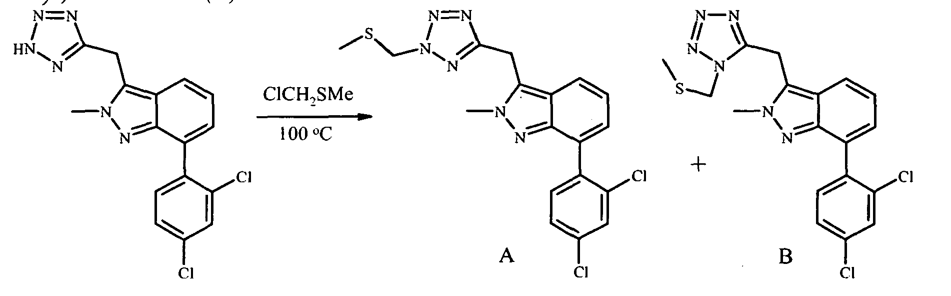

Tetrazole (349 mg, 0.97 mmol) in chloromethylmethylsulfide (3 mL) was stirred at 100°C for 2.5 hours and cooled to room temperature. The mixture was partitioned between EtOAc and water. The organic layer was dried (MgSO₄) and concentrated. The residue was purified on SiO₂, eluting with 35-60% EtOAc/hexanes to give product A (243 mg, 58%). Product B was in the other fractions which were retained and concentrated. This residue was then purified with preparative TLC (0.5 mm thickness, 20x20 cm², 60% EtOAc/hexanes) to give product B (46 mg, 11%).

### Synthesis of 7-(2,4-Dichloro-phenyl)-3-(2-methanesulfonylmethyl-2H-tetrazol-5-ylmethyl)-2-methyl-2H-indazole (I-12)

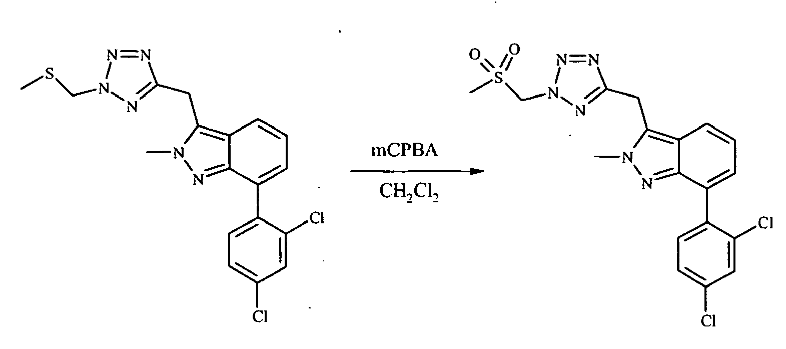

To a CH₂Cl₂ (3 mL) solution of sulfide (243 mg, 0.579 mmol) was added mCPBA (<77%, 365 mg, <2.1 mmol). The mixture was stirred at room temperature for 2 hours and partitioned between EtOAc and NaHCO₃ solution. The organic layer was washed with brine and dried (MgSO₄). The solvent was removed and the residue purified on SiO₂, eluting with 50-60% EtOAc/hexanes over 30 minutes to give the sulfone (160 mg, 61%).

### Example 5 (Method E)

### Synthesis of 2-{[7-(2,4-Dichlorophenyl)-2-methyl-2H-indazol-3-yl]-hydroxymethyl}-imidazole-1-sulfonic acid dimethylamide (I-35)

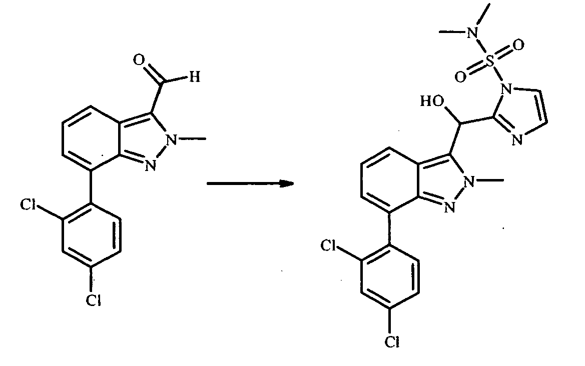

To a cooled (-78°C) solution of 1-(dimethylsulfamoyl)imidazole in dry THF (20 mL) was added 2 M BuLi (0.6 mL, 1.2 eq.) and stirred for 45 min. The solution was warmed to 0 °C for 10 min., cooled to -78°C, and a solution of 7-(2,4-dichloro-phenyl)-2-methyl-2H-indazole-3-carbaldehyde in dry THF (10 mL) was added to the reaction mixture which was allowed to warm to room temperature overnight. The reaction mixture was partitioned between water/brine and ethyl acetate, extracted with ethyl acetate, dried (Na₂SO₄)_{,} filtered, and solvent removed. The residue was purified on a silica gel column to give 400 mg of the product (82% yield).

### Synthesis of [7-(2,4-Dichlorophenyl)-2-methyl-2H-indazol-3-yl]-(1H-imidazol-2-yl)-methanol (I-28)

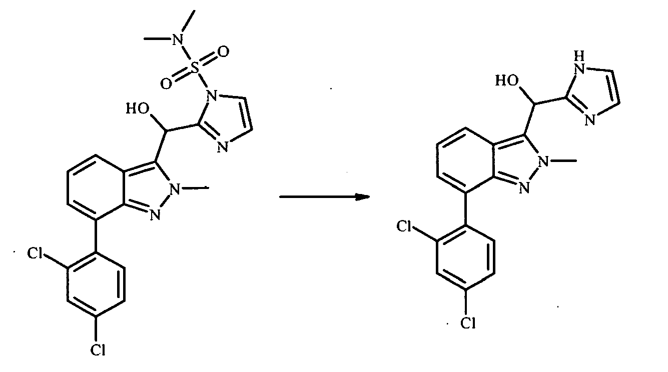

2-{[7-(2,4-Dichlorophenyl)-2-methyl-2H-indazol-3-yl]-hydroxymethyl}-imidazole-1-sulfonic acid dimethylamide (191 mg, 0.4 mmol) was dissolved in ethanol (14 mL), H₂O (12 mL), and H₂SO₄ (3 mL). This solution was stirred for 2.5 h at 80°C, poured into water, partially neutralized with NaHCO₃, and extracted with ethyl acetate. The extracts were dried (Na₂SO₄), filtered, solvent removed, and the residue purified on a silica gel column using methylene chloride/methanol to give 88 mg of product (60% yield).

### Synthesis of 7-(2,4-Dichlorophenyl)-3-(1H-imidazol-2-ylmethyl)-2-methyl-2H-indazole (I-21)

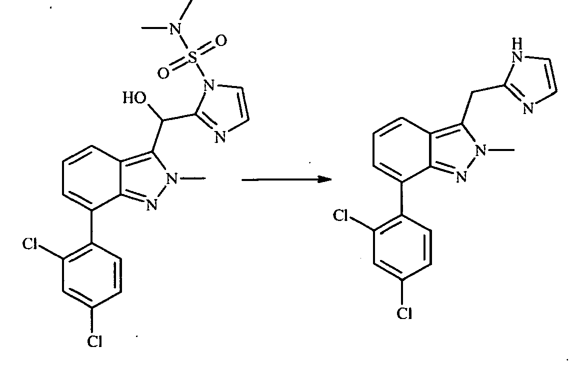

2-{ [7-(2,4-Dichlorophenyl)-2-methyl-2H-indazol-3-yl]-hydroxymethyl}-unidazole-1-sulfonic acid dimethylamide (80 mg, 0.2 mmol) was dissolved in a minimal amout of TFA and a few drops of triethylsilane were added. This mixture was heated to 70°C and stirred overnight. The reaction was basified with NaOH, extracted with ethyl acetate, and purified by HPLC.

### Synthesis of [7-(2,4-Dichlorophenyl)-2-methyl-2H-indazol-3-yl]-(1H-imidazol-2-yl)-methanone (I-30)

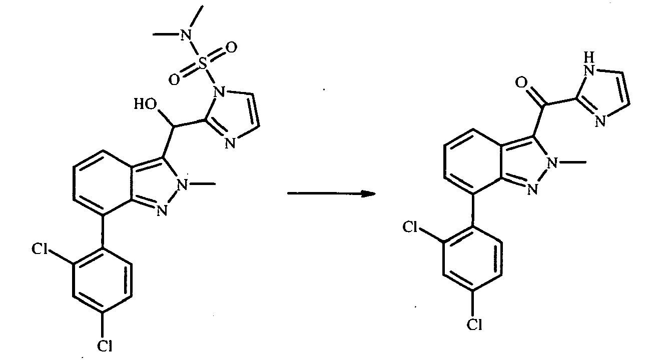

2-{[7-(2,4-Dichlorophenyl)-2-methyl-2H-indazol-3-yl)-hydroxymethyl}-imidazole-1-sulfonic acid dimethylamide (100 mg, 0.2 mmol) was added to a solution of ethanol (30 mL) and 10% NaOH (20 mL), and refluxed for 3 h. The reaction mixture was cooled, poured into water and extracted with methylene chloride. The extracts were dried (Na₂SO₄), filtered, solvent removed, and the residue purified on a silica gel column using methylene chloride to give 57 mg of the product (74% yield).

### Synthesis of [7-(2,4-Dichlorophenyl)-2-methyl-2H-indazol-3-yl]-[1-(2-hydroxyethyl)-1H-imidazol-2-yl]-methanone (I-24)

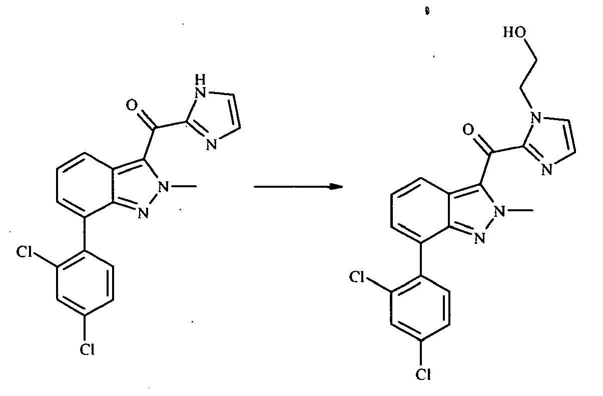

Ethylene carbonate (800 mg, 9 mmol) was stirred and heated to 100 °C, [7-(2,4-dichlorophenyl)-2-methyl-2H-indazol-3-yl]-(1H-imidazol-2-yl)-methanone (80 mg, 0.2 mmol) was added and the reaction stirred for 4 h. The reaction mixture was cooled adsorbed onto silica gel and the product eluted off to give 59 mg (66% yield).

### Example 6 (Method F)

### Synthesis of [7-(2,4-Dichlorophenyl)-2-methyl-2H-indazol-3-yl]-(3H-imidazol-4-yl)-methanol (I-27) and 7-(1,4-dichlorophenyl)-3-[ethoxy-(3H-imidazol-4-yl)-methyl]-2-methyl-2H-indazole (I-26)

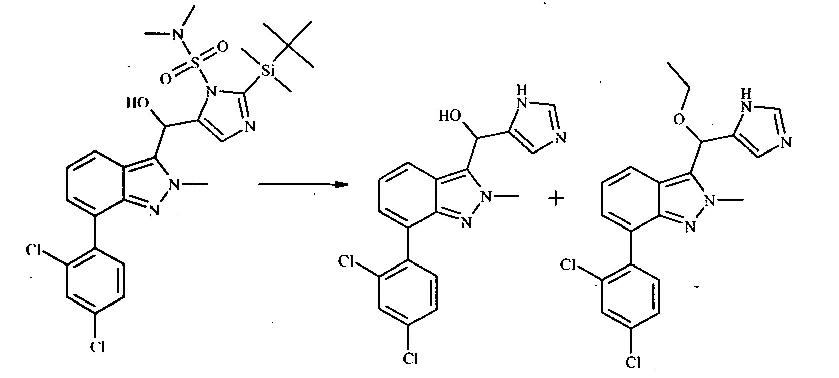

2-(t-Butyldimethylsilanyl)-5-{[7-(2,4-dichlorophenyl)-2-methyl-2H-indazol-3-yl]-hydroxymethyl}-imidazole-1-sulfonic acid dimethyl amide (287 mg, 0.5 mmol) was dissolved in a solution of ethanol (10 mL) and 3 M HCl (12 mL) and refluxed for 1 h. The reaction was allowed to cool, neutralized with NH₄OH poured into water, and the organics extracted with methylene chloride. The extracts were dried (Na₂SO₄), filtered, and the solvent removed. The residue was purified on a silica gel column using ethyl acetate/methylene chloride/methanol to provide the 35 mg of the alcohol (19% yield) and 60 mg of the ethyl ether (31% yield).

### Example 7 (Method G)

### Synthesis of [7-(2,4-dichloro-phenyl)-2-methyl-2H-indazol-3-yl]-(2-methoxymethyl-2H-[1,2,4] triazol-3-yl)-methanol

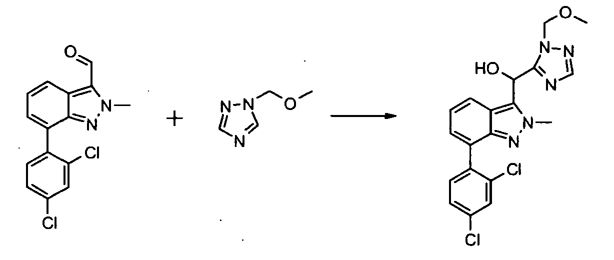

A solution of 1-methoxymethyl-1,2,4-triazole (82mg, 0.72 mmole) (prepared according to E. Regel, Liebigs Ann. Chem., 1977, 159) in 17 ml of dry THF under a nitrogen atmosphere was cooled to -78°C in a dry ice-acetone bath and added 0.46 ml of 1.6 M n-butyllithium in hexane. The solution was stirred for 1.5 hr and then 0.34 ml of HMPA was added followed by a solution of 7-(2,4-dichloro-phenyl)-2-methyl-2H-indazole-3-carbaldehyde (150 mg, 0.49 mmole) in 2 ml of dry THF. The solution was stirred at -78°C for 1 hr and then allowed to come to room temperature overnight. The reaction mixture was poured into 50 ml of saturated ammonium chloride solution, extracted with 3 x 30 ml of ethyl acetate, combined, washed with 50 ml of brine, dried over magnesium sulfate, and evaporated to dryness giving 302 mg of residue. Purification by flash column chromatography on silica gel using acetone/dichloromethane (1:4) afforded 62 mg (30% yield) of [7-(2,4-dichloro-phenyl)-2-methyl-2H-indazol-3-yl]-(2-methoxymethyl-2H-[1,2,4] triazol-3-yl)-methanol as a colorless oil, (M+H)⁺= 418.

### Synthesis of [7-(2,4-dichloro-phenyl)-2-methyl-2H-indazol-3-yl]-(2-methoxmethyl-2H-[1,2,4] triazol-3-yl)-methanone (I-19)

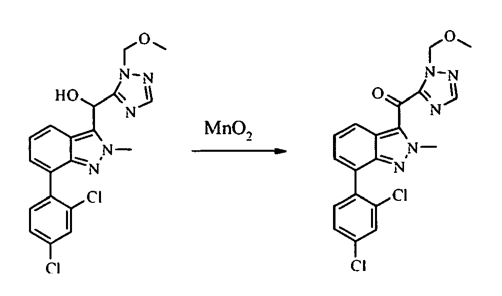

A solution of [7-(2,4-dichlorophenyl)-2-methyl-2H-indazol-3yl]-(2-methoxymethyl-2H-[1,2,4] triazol-3-yl)-methanol (62 mg, 0.15 mmole) in 4 ml of dichloromethane was treated with manganese dioxide (65 mg, 0.75 mmole) and the mixture was stirred at room temperature for 14 hrs. The mixture was filtered and the solvent evaporated. The residue was purified by flash column chromatography on silica gel eluting with ethyl acetate/hexane (1:4) giving 49 mg (80% yield) of [7-(2,4-dichloro-phenyl)-2-methyl-2H-indazol-3-yl]-(2-methoxymethyl-2H-[1,2,4] triazol-3-yl)-methanone as a yellow solid, (M+H)⁺= 416.

### Synthesis of [7-(2,4-dichloro-phenyl)-2-methyl-2H-indazol-3-yl]-(2H-[1,2,4]triazol-3-yl)-methanone (I-15)

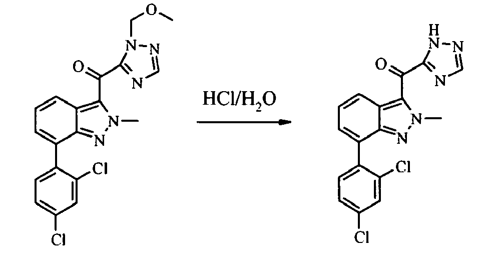

A mixture of [7-(2,4-dichloro-phenyl)-2-methyl-2H-indazol-3-yl]-(2-methoxymethyl-2H-[1,2,4] [triazol-3-yl)-methanone (40 mg, 0.096 mmole), 2 ml of methanol, and 2 ml of 6 M hydrochloric acid was heated to 90°C under a nitrogen atmosphere for 1 hr. After cooling the mixture was diluted with 25 ml of saturated sodium bicarbonate, filtered, washed with water, and dried. Purification by flash column on silica gel eluting with methanovdichloromethane (3:97) gave 22 mg of [7-(2,4-dichloro-phenyl)-2-methyl-2H-indazol-3-yl]-(2H-[1,2,4]triazol-3-yl)-methanone as a yellow solid, mp 284-285 °C.

### Example 8 (Method H)

### Synthesis of 7-(2,4-Dichlorophenyl)-3-(4,5-dihydro-1H-imidazol-2-ylmethyl)-2-methyl-2H-indazole (I-29)

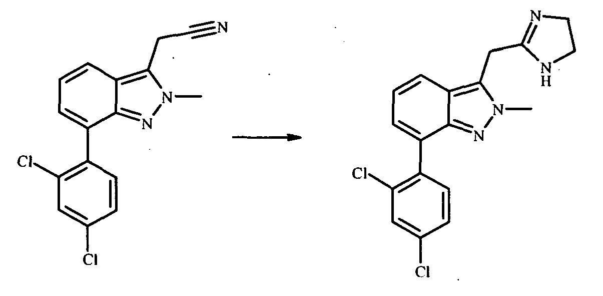

Phosphorus pentasulfide (16 mg, 3.5 mol %) and [7-(2,4-dichlorophenyl)-2-methyl-2H-indazol-3-yl]-acetonitrile were combined in ethylene diamine (5 mL) and stirred at 95°C for 4 h. The reaction was cooled, poured into water and extracted with ethyl acetate. The extracts were dried (Na₂SO₄), filtered, and the solvent removed. The residue was purified on a silica gel column using methylene chloride/methanol to give 291 mg of the product (81% yield).

### Example 9 (Method I)

### Synthesis of 7-(2,4-Dichloro-phenyl)-3-(1H-imidazol-4-yl)-2-methyl-2H-indazole (I-36)

A mixture of 7-(2,4-Dichloro-phenyl)-2-methyl-2H-indazole-3-carbaldehyde (120 mg, 0.393 mmol), tosylmethyl isocyanate (76.77 mg, 0.393 mmol) and potassium carbonate (54.35 mg, 3.393 mmol) in 1.5 ml methanol under an N₂ atmosphere was heated at reflux for 1 h. The reaction mixture was allowed to cool to room temperature, and the solvent was removed under reduced pressure. The residue was diluted with water and extracted with ethyl acetate (2x). The combined ethyl acetate extracts were dried over MgSO₄ Evaporation afforded 134.0 mg (99%) of 7-(2,4-Dichloro-phenyl)-2-methyl-3-oxazol-5-yl-2*H*-indazole (I-51) as a yellow solid, which was used without further purification.

7-(2,4-Dicbloro-phenyl)-2-methyl-3-oxazol-5-yl-2*H*-indazole (134.0 mg, 0.389 mmol) in 2.0 ml of formamide was heated in an oil bath under an N₂ atmosphere at 160°C overnight. The reaction mixture allowed to cool to room temperature and the precipitate was filtered off. The precipitate was taken up in ethyl acetate, washed with water then dried over MgSO₄, filtered and concentrated. Column chromatography (0 to 80% EtOAc/hexanes) afforded an impure solid.

The solid was dissolved in warm THF (1.0 ml) and treated with a 1.0 M HCl in ether solution. The resulting precipitate was filtered off and further purified through parallel synthesis to afford 7.49 mg of 7-(2,4-Dichloro-phenyl)-3-(1*H*-imidazol-4-yl)-2-methyl-2*H*-indazole.

### Example 10 (Method J)

### Synthesis of 7-(2,4-Dichloro-phenyl)-3-(1H-imidazol-4-yl)-2-methyl-2H-indazole (I-45)

To a solution of 7-(2,4-Dichloro-phenyl)-2-methyl-2*H*-indazole-3-carbaldehyde (80.0 mg, 0.262 mmol) in 2.0 ml of a 7.0 M ammonia in MeOH solution was added glyoxal (40% in water, 57.0 ul, 0.393 mmol) dropwise. The yellow suspension was stirred at room temperature for 6 days. Solvent was removed under reduced pressure and the residue was dissolved in water, then extracted with dichloromethane (3x). The combined dichloromethane extracts were dried over MgSO₄, filtered and concentrated. Column chromatography (0 to 60% AcOEt/Heptane) afforded 8.0 mg of a brown oil, which was slightly impure. The oil was further purified through parallel synthesis to afford 4.9 mg (5.45%) of 7-(2,4-Dichloro-phenyl)-3-(1*H*-imidazol-4-yl)-2-methyl-2H-indazole.

### Example 11 (Method K)

### Synthesis of N-[7-(2,4-dichloro-phenyl)-2-methyl-2H-indazol-3-yl]-formimidic acid methyl ester

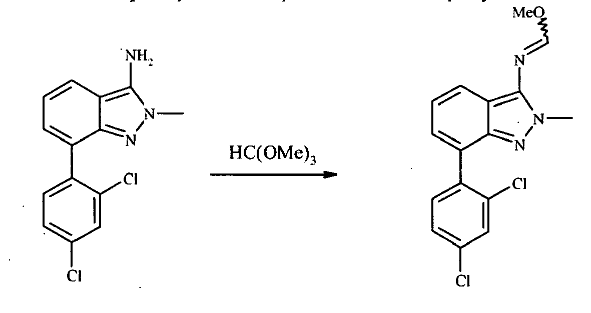

A mixture of 7-(2,4-dichloro-phenyl)-2-methyl-2H-indazol-3-ylamine (75 mg, 0.26 mmole), 5 ml of toluene, and 90 microliter of trimethylorthoformate was heated to 100°C for 5 hrs. The solvent was evaporated leaving 95 mg of crude N-[7-(2,4-dichloro-phenyl)-2-methyl-2H-indazol-3-yl]-formimidic acid methyl ester, (M+H)⁺ = 334.

### Synthesis of N-[7-(2,4-dichloro-phenyl)-2-methyl-2H-indazol-3-yl]-N'-(2,2-dimethoxy-ethyl)-formamidine

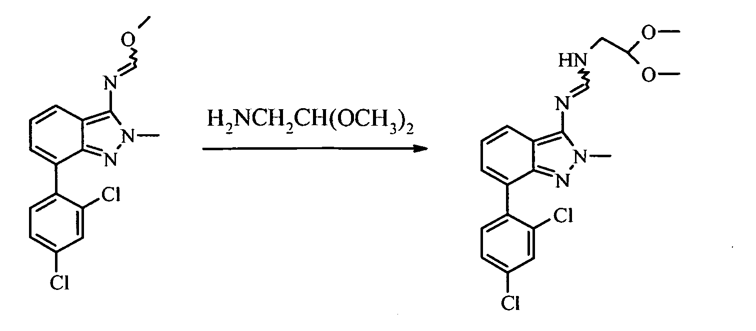

A mixture of N-[7-(2,4-dichloro-phenyl)-2-methyl-2H-indazol-3-yl]-formimidic acid methyl ester (85 mg, 0.25 mmole) dissolved in 8 ml of methanol and 0.19 ml (1.74 mmole) of 2-aminoacetaldehyde dimethyl acetal was heated at 85°C under a nitrogen atmosphere for 3 hrs. The solvent was evaporated giving 125 mg of crude N-[7-(2,4-dichloro-phenyl)-2-methyl-2H-indazol-3-yl]-N'-(2,2-dimethoxy-ethyl)-formamidine, (M=H)⁺= 407.

### Synthesis of 7-(2,4-dichloro-phenyl)-3-imidazol-1-yl-2-methyl-2H-indazole (I-22)

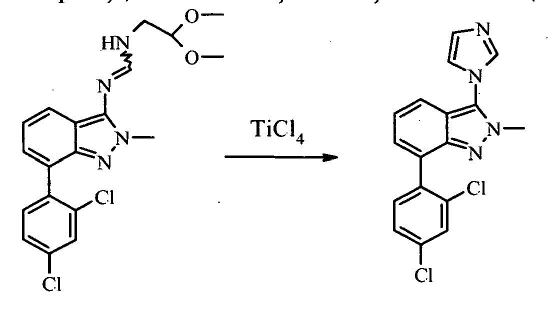

To a solution of N-[7-(2,4-Dichloro-phenyl)-2-methyl-2H-indazol-3-yl]-N'-(2,2-dimethoxyethyl)-formamidine (100mg, 0.25 mmole) in 2 ml of dimethoxyethane was added 0.34 ml (0.34 mmole) of 1 M titanium tetrachloride in dichloromethane and the brown mixture was heated to 100°C under a nitrogen atmosphere for 2 hrs. Cooled to room temperature, diluted with 50 ml of water, basified with 2.5M sodium hydroxide, extracted 3 x 30 ml of dichloromethane, combined, washed with brine, dried over magnesium sulfate, and evaporated to dryness. The residue was purified by flash column chromatography on silica gel eluting with methanol/dichloromethane (3:97) giving 53 mg (57% yield) of 7-(2,4-dichloro-phenyl)-3-imidazol-1-yl-2-methyl-2H-indazole, mp 63-89 °C.

### Example 12 (Method L)

### Synthesis of 7-(2,4-dichloro-phenyl)-2-methyl-3-[1,2,4]triazol-4-yl-2H-indazole (I-23)

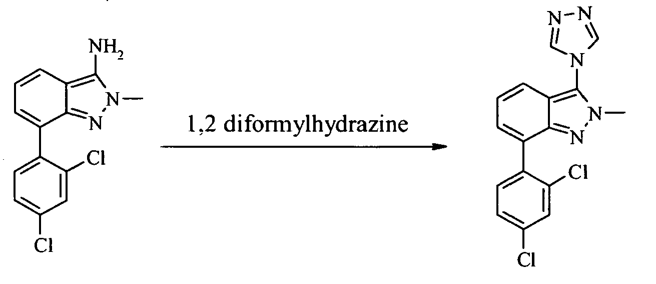

A mixture of 7-(2,4-dichloro-phenyl)-2-methyl-2H-indazol-3-ylamine (75 mg, 0.26 mmole) in 2 ml of dry pyridine was treated with 1,2-diformylhydrazine (68 mg, 0.77 mmole), 0.49 ml (3.9 mmole) of chloro-trimethylsilane, and then 0.25 ml (1.8 mmole) of triethylamine. The mixture was irradiated in the microwave reactor for 30 minutes at 150°C. The solvent was evaporated, the residue treated with 30 ml of water, extracted 2 x 25 ml of ethyl acetate. The organic layers were combined, washed with brine, dried over magnesium sulfate, and concentrated. The residue was purified by flash chromatography eluting with acetone/dichloromethane (1:4) giving 50 mg (56% yield) of 7-(2,4-dichloro-phenyl)-2-methyl-3-[1,2,4]triazol-4-yl-2H-indazole, mp 118-121 °C.

### Example 13 (Method M)

### Synthesis of [7-(2,4-dichloro-phenyl)-2-methyl-2H-indazol-3-yl]-acetaldehyde

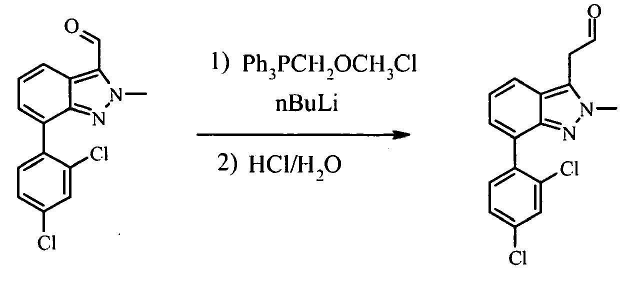

A suspension of (methoxymethyl)triphenylphosphonium chloride (494 mg, 1.44 mmole) in 10 ml of dry THF under a nitrogen atmosphere was cooled to -78°C and treated with 0.98 ml (1.57 mmole) of 1.6 M n-butyllithium in hexane and the brown solution was stirred for 10 minutes. A solution of 7-(2,4-dichloro-phenyl)-2-methyl-2H-indazole-3-carbaldehyde (0.40 g, 1.31 mmole) in 4 ml of dry THF was added and the resultant light brown solution was allowed to warm to room temperature overnight. After 14 hrs, the mixture was poured into 60 ml of saturated ammonium chloride and extracted with 2 x 50 ml of ethyl acetate. The organic layers were combined, washed with brine, dried over magnesium sulfate, and concentrated. The residue was purified by flash chromatography on silica and eluted with ethyl acetate/hexane (1:4) giving 203 mg (46% yield) of 7-(2,4-dichloro-phenyl)-3-(2-methoxy-vinyl)-2-methyl-2H-indazole as a yellow oil, (M + H)⁺ = 333. A solution of this intermediate (200 mg, 0.60 mmole) dissolved in 4 ml of THF was treated with 0.5 ml (1.0 mmole) of 2 M hydrochloric acid under a nitrogen atmosphere, and stirred at 80°C for 14 hrs. The solvent was evaporated, and the residue was diluted with 10 g of ice, 30 ml of saturated sodium bicarbonate, and extracted 2 x 25 ml of ethyl acetate. The organic layers were combined, washed with brine, dried over magnesium sulfate, and concentrated to give 190 mg of crude [7-(2,4-dichloro-phenyl)-2-methyl-2H-indazol-3-yl]-acetaldehyde as a yellow oil, (M + H)⁺ = 319.

### Synthesis of 7-(2,4-dichloro-phenyl)-2-methyl-3-[4-(toluene-4-sulfonyl)-4,5-dihydro-oxazol-5-ylmethy/]-2H-indazo/e

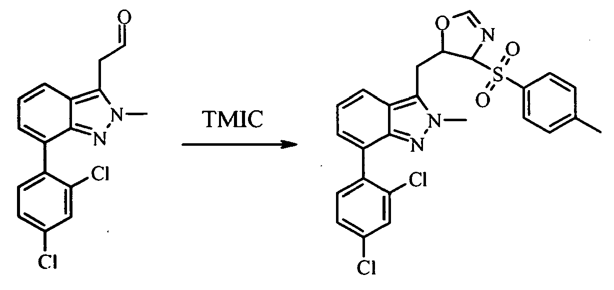

To a solution of [7-(2,4-dichloro-phenyl)-2-methyl-2H-indazol-3-yl]-acetaldehyde (180 mg, 0.56 mmole) in 4 ml of ethanol was cooled in an ice bath under a nitrogen atmosphere was added 110 mg (0.56 mmole) of tosylmethyl isocyanide and 3 mg of sodium cyanide. The heterogeneous yellow mixture was stirred and allowed to warm to room temperature overnight. The solvent was removed under reduced pressure. The residue was treated with 10 g of ice and 40 ml of saturated sodium bicarbonate and extracted 2 x 25 ml of ethyl acetate. The organic layers were combined, washed with brine, dried over magnesium sulfate, and concentrated. The residue was purified by flash column chromatography on silica gel eluting with ethyl acetate/hexane (1:3) giving 87 mg (30% yield) of 7-(2,4-dichloro-phenyl)-2-methyl-3-[4-(toluene-4-sulfonyl)-4,5-dihydro-oxazol-5-ylmethyl]-2H-indazole as a tan solid, (M + H)⁺ = 514.

### Synthesis of 7-(2,4-dichloro-phenyl)-3-(3H-imidazol-4-ylmethyl)-2-methyl-2H-indazole (I-25)

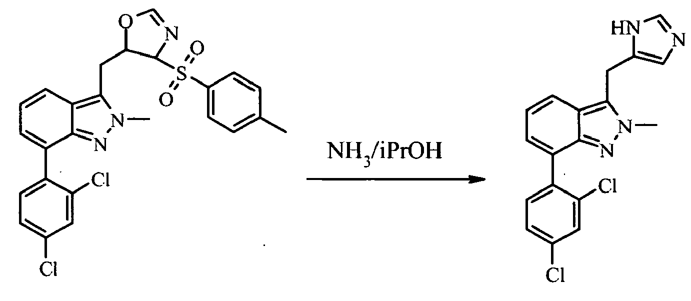

In a 15 ml thick-walled tube was placed 7-(2,4-dichloro-phenyl)-2-methyl-3-[4-(toluene-4-sulfonyl)-4,5-dihydro-oxazol-5-ylmethyl]-2H-indazole (77 mg, 0.15 mmole) dissolved in 5 ml of 2 M ammonia in isopropanol, cooled in an ice bath and bubbled in ammonia gas until saturated. The tube was sealed and heated to 100°C for 6 hrs. The solvent was evaporated and the residue was purified by flash chromatography on silica gel eluting with methanol/dichloromethane (5:95) giving 6 mg (10% yield) of 7-(2,4-dichloro-phenyl)-3-(3H-imidazol-4-ylmethyl)-2-methyl-2H-indazole as a dark solid, (M +H)⁺ = 357.

### Example 14 (Method N)

### Synthesis of 3-[7-(2,4-dichloro-phenyl)-2-methyl-2H-indazol-3-yl]-acrylonitrile

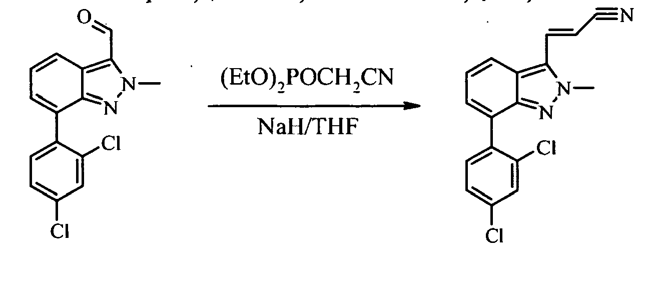

Sodium hydride (126 mg of 60% in mineral oil, 3.15 mmole) was washed with hexane and suspended in 20 ml of dry THF. The suspension was cooled in an ice bath under a nitrogen atmosphere. To this suspension was added dropwise 0.51 ml (0.557 g, 3.15 mmole) of diethyl(cyanomethyl)phosphonate. The ice bath was removed and the resulting solution was stirred at room temperature for 30 minutes, after which 800 mg (2.62 mmole) of 7-(2,4-dichloro-phenyl)-2-methyl-2H-indazole-3-carbaldehyde in 20 ml of dry THF was added, and the resulting mixture was stirred for 14 hrs. The reaction mixture was poured into 50 ml of saturated ammonium chloride, diluted with 100 ml of water, and extracted with 150 ml of ethyl acetate and with 75 ml of dichloromethane. The organic layers were combined, washed with brine, dried over magnesium sulfate, and concentrated to afford 866 mg (90% yield) of 3-[7-(2,4-dichloro-phenyl)-2-methyl-2H-indazol-3-yl]-acrylonitrile as a yellow solid, mp 258-259 °C (ethyl acetate).

### Synthesis of 3-[7-(2,4-dichloro-phenyl)-2-methyl-2H-indazol-3-yl]-propionitrile

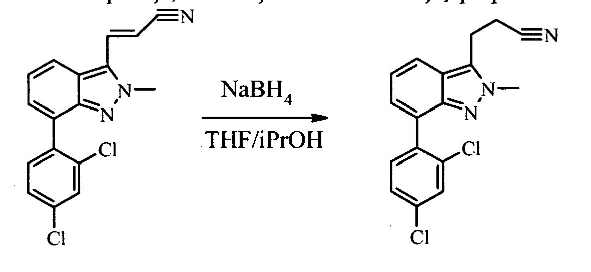

A suspension of 100 mg (0.305 mmole) of 3-[7-(2,4-dichloro-phenyl)-2-methyl-2H-indazol-3-yl]-acrylonitrile in 4 ml of THF and 4 ml of isopropanol was treated with 46 mg (1.22 mmole) of sodium borohydride, and the resulting suspension was heated to 100°C for 4 hrs. The solvent was removed. The residue was diluted with 50 ml of water and the pH was adjusted to pH 6 with 1M hydrochloric acid. The mixture was extracted with 50 ml of ethyl acetate and 25 ml of dichloromethane. The organic layers were combined, washed with brine, dried over magnesium sulfate, and concentrated. The residue was purified by flash chromatography eluting with ethyl acetate/hexane (3:7) to provide 40 mg (40% yield) of 3-[7-(2,4-dichloro-phenyl)-2-methyl-2H-indazol-3-yl]-propionitrile as a white solid, mp 192-193 °C.

### Example 15 (Method O)

### Synthesis of 7-(2,4-dichloro-phenyl)-2-methyl-3-[2-(2H-[1,2,4]triazol-3-yl)-ethyl]-2H-indazole (I-34)

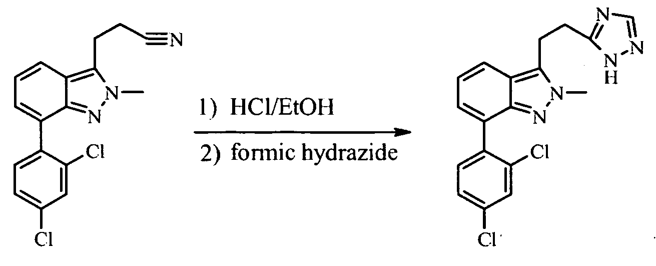

A solution of 200 mg (0.605 mmole) of 3-[7-(2,4-dichloro-phenyl)-2-methyl-2H-indazol-3-yl]-propionitrile in 6 ml of dichloromethane and 74 microliters (58 mg, 1.26 mmole) of abs. ethanol was cooled in an ice bath under a nitrogen atmosphere. Hydrogen chloride gas was bubbled in for about 5 minutes or until the solution was saturated. The flask was tightly stoppered and stored at 5 °C for 4 days. The crystals were quickly filtered and briefly dried leaving 240 mg of ethyl imidate salt.

A mixture of 36 mg (0.6 mmole) of formic hydrazide in 4 ml of dry pyridine was treated with 0.2 g of powdered molecular 4A sieve and was stirred at room temperature for 30 minutes. This mixture was transferred to a flask containing 240 mg(0.6 mmole) of the above imidate salt and the heterogeneous mixture was stirred under a nitrogen atmosphere at room temperature for 14 hrs and then heated to 90°C for 16 hrs. The solvent was evaporated and the residue was treated with 40 ml of ethyl acetate and 25 ml of saturated sodium bicarbonate solution, and the mixture was filtered to remove the molecular sieve. The filtrate was separated and the organic layer was washed with brine, dried over magnesium sulfate, and concentrated. The residue was purified by flash chromatography eluting with methanol/dichloromethane (5:95) to provide 69 mg (30% yield) of 7-(2,4-dichloro-phenyl)-2-methyl-3-[2-(2H-[1,2,4]triazol-3-yl)-ethyl]-2H-indazole as a white solid, mp 92-116 °C.

### Example 16 (Method P)

### Synthesis of 7-(2,4-dichloro-phenyl)-2-methyl-2H-indazole-3-carboxylic acid hydrazide

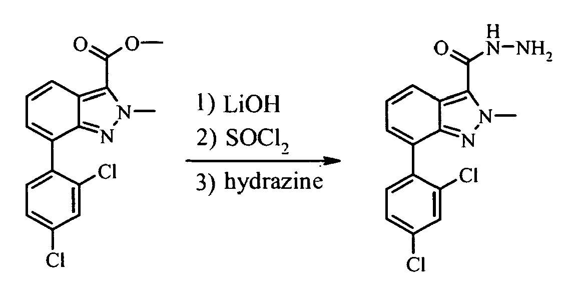

A mixture of 85 mg (0.25 mmole) of 7-(2,4-dichloro-phenyl)-2-methyl-2H-indazole-3-carboxylic acid methyl ester in 3 ml of methanol and 16 mg (0.38 mmole) of lithium hydroxide monohydrate in 1 ml of water was heated to 90°C under a nitrogen atmosphere for 3 hrs, then cooled to room temperature and acidified to precipitate the product. The mixture was filtered, washed with water, and dried under reduced pressure to give 77 mg (90% yield) of the acid product, (M+H)⁺ = 321.

The acid (75 mg, 0.23 mmole) was suspended in 3 ml of chloroform, added 0.050 ml (83 mg, 0.70 mmole) of thionyl chloride, a drop of DMF, and placed under a nitrogen atmosphere and heated to 70°C for 16 hrs. The solvent was evaporated and the residue was further azeotroped with toluene and dried under vacuum to obtain 79 mg of the acid chloride product.

To a 0 °C solution of the acid chloride (75 mg, 0.23 mmole) in 3 ml of dichloromethane was added 0.098 ml (0.7 mmole) of triethylamine and 0.037 ml (37 mg, 1.2 mmole) of anydrous hydrazine. The ice-bath was removed and the reaction mixture was stirred at room temperature for 20 hrs and diluted with 40 ml of dichloromethane. The resulting mixture was washed with 40 ml of water. The organic layer was dried over magnesium sulfate, and concentrated. The residue was purified by flash chromatography using methanol/dichloromethane (5:95) to give 61 mg (78% yield) of 7-(2,4-dichloro-phenyl)-2-methyl-2H-indazole-3-carboxylic acid hydrazide as a white solid, (M +H)⁺ = 335.

### Synthesis of 7-(2,4-dichloro-phenyl)-2-methyl-3-[1,3,4]oxadiazol-2-yl-2H-indazole (I-39)

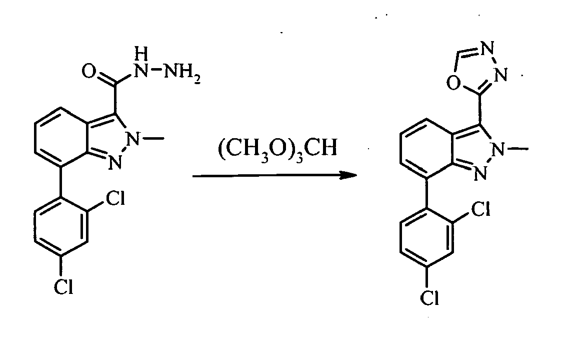

A mixture of 84 mg (0.25 mmole) of 7-(2,4-dichloro-phenyl)-2-methyl-2H-indazole-3-carboxylic acid hydrazide, 1 ml (0.97 g, 9.1 mmole) of trimethylorthoformate, and 48 mg (0.25 mmole) of p-toluenesulfonic acid mono-hydrate was placed under a nitrogen atmosphere and heated to 115°C for 10 hrs. The solvent was evaporated, the residue was taken up in 50 ml of ethyl acetate, washed with 30 ml of saturated sodium bicarbonate and 30 ml of brine. The organic layer was dried over magnesium sulfate, and concentrated. The residue was purified by flash chromatography eluting with ethyl acetate/hexane (5:95) to yield 34mg (39% yield) of 7-(2,4-dichloro-phenyl)-2-methyl-3-[1,3,4]oxadiazol-2-yl-2H-indazole as a white solid, mp 199-201°C.

### Example 17 (Method Q)

### Synthesis of 7-(2,4-dichloro-phenyl)-3-(4,5-dihydro-oxazol-2-yl)-2-methyl-2H-indazole (I-48)

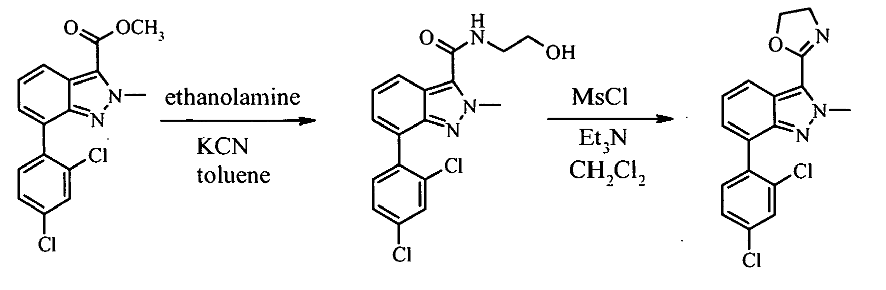

A solution of 150 mg (0.448 mmole) of 7-(2,4-dichloro-phenyl)-2-methyl-2H-indazole-3-carboxylic acid methyl ester in 3 ml of toluene was treated with 6 mg of potassium cyanide and 35 microliter (36 mg, 0.58 mmole) of ethanolamine. The mixture was heated to 130°C for 90 minutes. The solvent was evaporated and the residue was treated with 40 ml of water, mixed thoroughly, filtered, and vacuum dried to give 161 mg of 7-(2,4-dichloro-phenyl)-2-methyl-2H-indazole-3-carboxylic acid (2-hydroxy-ethyl)-amide as a white solid, mp 188-190 °C. This intermediate (140 mg, 0.38 mmole) was dissolved in 5 ml of dichloromethane and 0.54 ml (388 mg, 3.84 mmole) of triethylamine, placed under a nitrogen atmosphere, cooled in an ice bath, treated with 37 microliter (55 mg, 0.48 mmole) of methanesulfonyl chloride. The mixture was allowed to slowly warm to room temperature overnight. After 14 hrs, the mixture was heated to 60°C for 16 hrs, then cooled to room temperature, and diluted with 30 ml of saturated sodium bicarbonate solution. The aqueous layer was separated and extracted with 2 x 30 ml of dichloromethane. The organic layers were combined, washed with brine, dried over magnesium sulfate, and concentrated. The residue was purified by flash chromatography eluting with ethyl acetate/hexane (1:4) to afford 59 mg (39% yield) of 7-(2,4-dichloro-phenyl)-3-(4,5-dihydro-oxazol-2-yl)-2-methyl-2H-indazole as a white solid, mp 162-167 °C.

### Example 18 (Method R)

### Synthesis of 7-(2,4-dichloro-phenyl)-2-methyl-3-(3-methyl-[1,2,4]oxadiazol-5-yl)-2H-indazole (I-50)

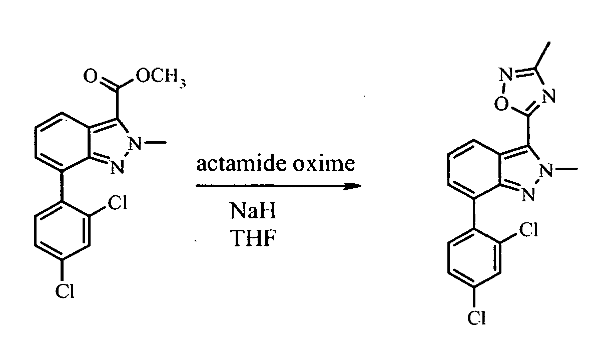

To a mixture of 14 mg (0.36 mmole) of 60% sodium hydride in mineral oil, 27 mg (0.36 mmole) of acetamide oxime, and 100 mg of powdered 4Å molecular sieve was added 4 ml of dry THF. The mixture was placed under a nitrogen atmosphere, heated to 60°C for 1 hr. To the resulting mixture was then added a solution of 100 mg (0.30 mmole) of 7-(2,4-dichloro-phenyl)-2-methyl-2H-indazole-3-carboxylic acid methyl ester in 2 ml of dry THF. The resulting mixture was then heated to reflux at 85°C for 3 hrs, cooled to room temperature, filtered, and the solid was washed with 30 ml of ethyl acetate. The filtrate was washed with brine, dried over magnesium sulfate, and concentrated. The residue was purified by flash chromatography eluting with ethyl acetate/hexane (I:9) to give 74 mg (69% yield) of 7-(2,4-dichloro-phenyl)-2-methyl-3-(3-methyl-[1,2,4]oxadiazol-5-yl)-2H-indazole as a white solid, mp 178-178.5 °C.

### Example 19

Pharmaceutical preparations for delivery by various routes are formulated as shown in the following Tables. "Active ingredient" or "Active compound" as used in the Tables means one or more of the Compounds of Formula I.

| Composition for Oral Administration | |
|---|---|
| **Ingredient** | **% wt./wt.** |
| Active ingredient | 20.0% |
| Lactose | 79.5% |
| Magnesium stearate | 0.5% |

The ingredients arc mixed and dispensed into capsules containing about 100 mg each; one capsule would approximate a total daily dosage.

| Composition for Oral Administration | |
|---|---|
| **Ingredient** | **% wt./wt.** |
| Active ingredient | 20.0% |
| Magnesium stearate | 0.5% |
| Crosscarmellose sodium | 2.0% |
| Lactose | 76.5% |
| PVP (polyvinylpyrrolidine) | 1.0% |

The ingredients are combined and granulated using a solvent such as methanol. The formulation is then dried and formed into tablets (containing about 20 mg of active compound) with an appropriate tablet machine.

| Composition for Oral Administration | |
|---|---|
| **Ingredient** | **Amount** |
| Active compound | 1.0 g |
| Fumaric acid | 0.5 g |
| Sodium chloride | 2.0 g |
| Methyl paraben | 0.15 g |
| Propyl paraben | 0.05 g |
| Granulated sugar | 25.5 g |
| Sorbitol (70% solution) | 12.85 g |
| Veegum K (Vanderbilt Co.) | 1.0 g |
| Flavoring | 0.035 ml |
| Colorings | 0.5 mg |
| Distilled water | q.s. to 100 ml |

The ingredients are mixed to form a suspension for oral administration.

| Parenteral Formulation | |
|---|---|
| **Ingredient** | **% wt./wt.** |
| Active ingredient | 0.25 g |
| Sodium Chloride | qs to make isotonic |
| Water for injection | 100 ml |

The active ingredient is dissolved in a portion of the water for injection. A sufficient quantity of sodium chloride is then added with stirring to make the solution isotonic. The solution is made up to weight with the remainder of the water for injection, filtered through a 0.2 micron membrane filter and packaged under sterile conditions.

| Suppository Formulation | |
|---|---|
| **Ingredient** | **% wt./wt**. |
| Active ingredient | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

| Topical Formulation | |
|---|---|
| **Ingredients** | **grams** |
| Active compound | 0.2-2 |
| Span 60 | 2 |
| Tween 60 | 2 |
| Mineral oil | 5 |
| Petrolatum | 10 |
| Methyl paraben | 0.15 |
| Propyl paraben | 0.05 |
| BHA (butylated hydroxy anisole) | 0.01 |
| Water | q.s. 100 |

All of the ingredients, except water, are combined and heated to about 60°C with stirring. A sufficient quantity of water at about 60°C is then added with vigorous stirring to emulsify the ingredients, and water then added q.s. about 100 g.

### Nasal Spray Formulations

Several aqueous suspensions containing from about 0.025-0.5 percent active compound are prepared as nasal spray formulations. The formulations optionally contain inactive ingredients such as, for example, microcrystalline cellulose, sodium carboxymethylcellulose, dextrose, and the like. Hydrochloric acid may be added to adjust pH. The nasal spray formulations may be delivered via a nasal spray metered pump typically delivering about 50-100 microliters of formulation per actuation. A typical dosing schedule is 2-4 sprays every 4-12 hours.

### Example 20

### 35S-TBPS binding assay

The binding assay is based on the assay reported by K. Gee et al., Eur. J. Pharmacol. 1987, 136, 419-423.

Homogenate preparation: Membrane preparations of HEK293 cells containing either GABA_{A} α1β2γ2 or GABA_{A} α2β3γ2 constructs were performed according to a modified procedure previously described by Gee et al. (*supra*)*.* Whole HEK 293 cells in D-PBS (calcium/magnesium free) buffer adjusted to pH 7.4 were centrifuged at 7,280 x g for 20 m. After discarding the supernatant, the pellet was resuspended in the buffer and centrifuged at 1,820 x g for 10 m. Afterwards, the supernatant was discarded and the pellet resuspended in ice-cold preparation buffer (50 mM Tris HCl pH 7.4, 4 °C and 150 mM KCl), homogenized for 30 sec using a Brinkmann Polytron PT3000 (setting 6) and centrifuged at 48,000 x g for 30 m at 4 °C. The centrifugation and homogenization procedure was repeated two more times for a total of 3 times before resuspending the membranes at a final protein concentration of 0.5 mg/mL. Aliquots (30 mL) of the final membrane preparation were then centrifuged at 48,000 x g for 30 m, and the resulting pellets were stored at -80 °C until required.

35S-TBPS binding assay. Membrane pellets containing either GABA_{A} α1β2γ2 or GABA_{A} α2β3γ2 constructs were thawed on ice, resuspended in 10 mL of 50 mM Tris HCl pH7.4, 4 °C and 150 mM KCl and centrifuged at 48,000xg, 30 m at 4 °C. After discarding the supernatant, the pellet was resuspended in 30 mL incubation buffer (50 mM Tris HCl pH 7.4, 25°C and 150 mM KCl) at approximately 0.5 mg/mL protein concentration. In 35S-TBPS competition studies, HEK293 membranes were incubated with 35S-TBPS (5 nM final) and GABA (1 µM) in the absence or presence of competitor at concentrations ranging from 0.01 nM to 10 µM in 125 µL incubation buffer for 2 hours at room temperature (~22°C). Non-specific binding was assayed with picrotoxin (100 µM final concentration). The binding reaction was terminated by vacuum filtration through GF/B filters previously soaked in 0.1 % polyethylenimine followed by 3x1 mL washes with ice cold wash buffer (50 mM Tris HCl pH7.4, 4 °C and 150 mM KCI). Measurement of bound radioactivity was performed using a Packard Microplate 96 well topcount scintillation counter. Analysis of competition curves and estimation of pIC₅₀ values of test compounds were performed using the software programs ActivityBase and/or Prism (version 3.0). Some of the representative 35S-TBPS binding assay results are shown in Table 2 below.

| TABLE 2 | | | | | | | | | |
|---|---|---|---|---|---|---|---|---|---|
| Cpd | | α1β2γ2 | | α2β2γ2 | Cpd | | α1β2γ2 | | α2β2γ2 |
| | | | | | I-31 | | 5.38 | | 6.45 |
| I-7 | < | 5.00 | | 5.57 | I-32 | | 5.23 | | 6.40 |
| I-9 | < | 5.00 | < | 5.00 | 1-33 | | 5.83 | | 6.99 |
| I-10 | < | 5.00 | | 6.40 | I-34 | | 5.02 | | 6.51 |
| I-11 | | 5.82 | | 6.46 | I-35 | < | 5.00 | | |
| I-12 | | 5.94 | | 6.57 | I-36 | | 5.14 | | 6.84 |
| I-13 | | | | 6.04 | I-37 | | 5.56 | | 6.42 |
| I-14 | | 5.32 | | 6.08 | I-38 | < | 5.00 | < | 5.00 |
| I-15 | < | 5.00 | | 6.48 | I-39 | < | 5.00 | | 5.55 |
| I-16 | | 5.23 | | 5.92 | I-40 | | 5.39 | | 5.94 |
| I-17 | < | 5.00 | | 6.37 | | | | | |
| I-18 | | 6.03 | | 6.96 | I-42 | | 5.30 | | 6.22 |
| I-19 | < | 5.00 | | 5.79 | I-43 | | 5.05 | | 6.08 |
| I-20 | | | | 5.77 | | | | | |
| I-21 | | 5.45 | | 6.26 | I-45 | | 5.25 | | 6.27 |
| I-22 | | 5.50 | | 6.45 | I-46 | | 6.32 | | 6.22 |
| I-23 | < | 5.00 | | 5.39 | I-47 | | 5.92 | | 6.82 |
| I-24 | | 5.43 | | 6.67 | | | | | |
| I-25 | < | 5.00 | | 5.94 | I-49 | | 5.65 | | 6.43 |
| I-26 | | 5.21 | | 6.29 | I-50 | < | 5.00 | | 6.17 |
| I-27 | | 5.63 | | 6.26 | I-51 | | 5.52 | | 6.78 |
| I-28 | | 6.24 | | 6.83 | I-52 | | 5.44 | | 6.85 |
| I-29 | < | 5.00 | < | 5.00 | I-53 | | 5.65 | | 6.63 |
| I-30 | | 5.35 | | 6.78 | I-54 | | 5.53 | | 6.16 |

The foregoing discussion of the invention has been presented for purposes of illustration and description. The foregoing is not intended to limit the invention to the form or forms disclosed herein. Although the description of the invention has included description of one or more embodiments and certain variations and modifications, other variations and modifications are within the scope of the invention, e.g., as may be within the skill and knowledge of those in the art, after understanding the present disclosure. It is intended to obtain rights which include alternative embodiments to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

## Claims

1. A compound of the formula: 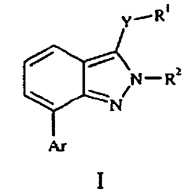 or a pharmaceutically acceptable salt thereof,
wherein:
Y is (CH₂)ₙ, alkenylene, CH(OR³) or C=O;
R¹ is a five-membered heteroaryl containing at least one nitrogen ring atom, wherein said five-membered heteroaryl ring is optionally substituted with one or more substituents each of which is independently selected from the group consisting of C₁₋₆ alkyl, C₁₋₆ heteroalkyl, -(CH₂)ₘCOX¹ -(CH₂)ₘSO₂X², and oxo;
X¹ is -OR³ or -NR⁴R⁵;
X² is C₁₋₆ alkyl or -NR⁴R⁵;
R² is hydrogen, C₁₋₆ alkyl, C₁₋₆ haloalkyl or C₃₋₆ cycloalkyl;
Ar is aryl or heteroaryl, each optionally substituted with one or more substituents independently selected from the group consisting of C₁₋₆alkyl, C₁₋₆alkoxy, C₁₋₆ alkylthio, C₁₋₆alkylsulfonyl, -SO₂NR⁴R⁵, halogen, C₁₋₆ haloalkyl, cyano, nitro, and -NR⁶R⁷;
each of R⁶ and R⁷ is independently selected from the group consisting of hydrogen, C₁₋₉ alkyl, and C₁₋₉ alkylcarbonyl;
each of R³, R⁴, and R⁵ is independently hydrogen or C₁₋₆ alkyl;
m is an integer from 0 to 4; and
n is an integer from 0 to 3.

2. The compound according to Claim 1, wherein R¹ is triazolyl, imidazolyl, tetrazolyl, pyrazolyl, or oxadiazolyl, each of which is optionally substituted.

3. The compound according to Claim 2, wherein R¹ is [1,2,4]triazol-1-yl, imidazol-1-yl, tetrazol-2-yl, tetrazol-1-yl, oxazolidin-1-yl, tetrazol-5-yl, [1,2,4]triazol-3-yl, [1,2,3,]triazol-1-yl, [1,2,3,]triazol-2-yl, imidazol-2-yl, [1,2,4]triazol-4-yl, imidazol-4-yl, pyrazol-1-yl, [1,3,4]oxadiazol-2-yl, [1,2,4]oxadiazol-5-yl, oxazol-5-yl, or [1,2,4]triazol-2-yl, each of which is optionally substituted.

4. The compound according to Claim 3, wherein R¹ is 5-carbamyl-2H-[1,2,4]triazol-1-yl; 5-carbamyl-1H-imidazol-1-yl; 3-carbmethoxyl-1H-[1,2,4]triazol-1-yl; 5-carbmethoxy-1H-[1,2,4]triazol-1-yl; 5-carbethoxymethyl-2H-tetrazol-2-yl; 5-carbethoxymethyl-1H-tetrazol-1-yl; 1-methanesulfonylmethyl-1H-tetrazol-5-yl; 1-(2-hydroxylethyl)-1H-tetrazol-5-yl; 2-(2-hydroxylethyl)-2H-tetrazol-5-yl; 2-methanesulfonylmethyl-2H-tetrazol-5-yl; 5-carbmethoxy-1H-imidazol-1-yl; 5-carbethoxy-1H-imidazol-1-yl; 2H-[1,2,4]triazol-3-yl; 1H-[1,2,3]triazol-1-yl; 2H-[1,2,3]triazol-2-yl; 1H-tetrazol-5-yl; 2-methoxymethyl-2H-[1,2,4]triazol-3-yl; 1-methyl-1H-tetrazol-5-yl; 1H-imidazol-2-yl; 1H-imidazol-1-yl; 4H-[1,2,4]triazol-4-yl; 1-(2-hydroxyethyl)-1H-imidazol-2-yl; 3H-imidazol-4-yl; 1H-[1,2,4]triazol-3-yl; 1-methyl-1H-tetrazol-5-yl; 2-methyl-2H-tetrazol-5-yl; 2H-[1,2,4]triazol-3-yl; 1-(N,N-dimethyl sulfonamide)-1H-imidazol-2-yl; 1H-pyrazol-1-yl; [1.3,4]-oxadiazol-2-yl; 1H-[1,2,4]triazol-1-yl; 1H-tetrazol-1-yl; 1H-tetrazol-5-yl; 2H-tetrazol-2-yl; 3-methyl-[1,2,4]oxadiazol-5-yl; oxazol-5-yl; 5-carbamylmethyl-1H-tetrazol-1-yl; 3-carbamyl-1H-[1,2,4]triazol-1-yl; 3-hydroxymethyl-2H-[1,2,4]triazol-2-yl; 3-carbethoxymethyl-1H-[1,2,4]triazol-1-yl; 3-carbethoxymethyl-2H-[1,2,4]triazol-2-yl; or 5-(2-hydroxyethyl)-1H-tetrazol-1-yl.

5. The compound according to Claim 1, wherein Y is (CH₂)ₙ.

6. The compound according to Claim 5, wherein n is 0.

7. The compound according to Claim 5, wherein n is 1.

8. The compound according to Claim 5, wherein n is 2.

9. The compound according to Claim 1, wherein Y is C=O.

10. The compound according to Claim 1, wherein Y is CH(OR³).

11. The compound according to Claim 10, wherein R³ is H, methyl, or ethyl.

12. The compound according to Claim 1, wherein Y is alkenylene.

13. The compound according to Claim 12, wherein Y is ethenylene.

14. The compound according to Claim 1, wherein R² is C₁₋₆ alkyl.

15. The compound according to Claim 14, wherein R² is methyl.

16. The compound according to Claim 1, wherein Ar is disubstituted aryl or disubstituted heteroaryl.

17. The compound according to Claim 16, wherein Ar is disubstituted aryl.

18. The compound according to Claim 17, wherein Ar is disubstituted phenyl.

19. The compound according to Claim 18, wherein Ar is dihalide substituted phenyl, and wherein each halide is independently selected.

20. The compound according to Claim 19, wherein Ar is 2,4-dichlorophenyl.

21. The use of a compound of formula I 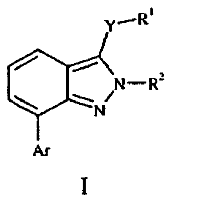 or a pharmaceutically acceptable salt thereof,
wherein:
Y is (CH₂)ₙ, alkenylene, CH(OR³) or C=O;
R¹ is a five-membered heteroaryl containing at least one nitrogen ring atom, wherein said five-membered heteroaryl ring is optionally substituted with one or more substituents each of which is independently selected from the group consisting of C₁₋₆ alkyl, C₁₋₆ heteroalkyl, -(CH₂)ₘCOX¹, -(CH₂)ₘSO₂X², and oxo;
X¹ is -OR³ or -NR⁴R⁵;
X² is C₁₋₆ alkyl or -NR⁴R⁵;
R² is hydrogen, C₁₋₆ alkyl, C₁₋₆ haloalkyl or C₃₋₆ cycloalkyl;
Ar is aryl or heteroaryl, each optionally substituted with one or more substituents independently selected from the group consisting of C₁₋₆ alkyl, C₁₋₆ alkoxy, C₁₋₆ alkylthio, C₁₋₆ alkylsulfonyl, -SO₂NR⁴R⁵ halogen, C₁₋₆ haloalkyl, cyano, nitro, and -NR⁶R⁷;
each of R⁶ and R⁷ is independently selected from the group consisting of hydrogen, C₁₋₉ alkyl, and C₁₋₉ alkylcarbonyl;
each of R³, R⁴ and R⁵ is independently hydrogen or C₁₋₆ alkyl;
m is an integer from 0 to 4; and
n is an integer from 0 to 3.
for the preparation of a medicament for the prevention or treatment of disorders alleviated by a positive allosteric modulator of a GABA_{A} receptor.

22. The use according to claim 21 wherein said disorder is depression, an anxiety disorder, a psychiatric disorder, a learning or cognitive disorder, a sleep disorder, a convulsive or seizure disorder or pain.

23. The use according to claim 22 wherein said compound is administered in combination with a selective serotonin reuptake inhibitor, a corticotropin releasing factor antagonist, or a phosphodiesterase IV inhibitor.

24. The use according to claim 21 wherein said positive allosteric modulator of a GABA_{A} receptor is a selective modulator of the α2 subtypes with respect to the all subtype.

25. A pharmaceutical composition for preventing or treating disorders alleviated by a positive allosteric modulator of a GABA_{A} receptor said composition comprising a therapeutically effective amount of a compound of claim 1 admixed with at least one diluent, excipient or carrier.

## Patentansprüche

1. Verbindung der Formel: 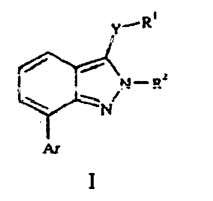 oder ein pharmazeutisch akzeptables Salz davon,
worin:
Y (CH₂)ₙ, Alkenylen, CH(OR³) oder C=O ist;
R¹ ein fünfgliedriges Heteroaryl, enthaltend mindestens ein Stickstoffringatom, ist, wobei der fünfgliedrige Heteroarylring gegebenenfalls mit einem oder mehreren Substituenten substituiert ist, von denen jeder unabhängig ausgewählt ist aus der Gruppe, bestehend aus C₁₋₆-Alkyl, C₁₋₆-Heteroalkyl, -(CH₂)ₘCOX¹, -(CH₂)ₘSO₂X² und Oxo;
X¹ -OR³ oder -NR⁴R⁵ ist;
X² C₁₋₆-Alkyl oder -NR⁴R⁵ ist;
R² Wasserstoff, C₁₋₆-Alkyl, C₁₋₆-Halogenalkyl oder C₃₋₆-Cyloalkyl ist;
Ar Aryl oder Heteroaryl ist, die jeweils gegebenenfalls mit einem oder mehreren Substituenten, unabhängig ausgewählt aus der Gruppe, bestehend aus C₁₋₆-Alkyl, C₁₋₆-Alkoxy, C₁₋₆-Alkylthio, C₁₋₆-Alkylsulfonyl, -SO₂NR⁴R⁵, Halogen, C₁₋₆-Halogenalkyl, Cyano, Nitro und -NR⁶R⁷, substituiert sind;
jeder von R⁶ und R⁷ unabhängig ausgewählt ist aus der Gruppe, bestehend aus Wasserstoff, C₁₋₉-Alkyl und C₁₋₉-Alkylcarbonyl,
jeder von R³, R⁴ und R⁵ unabhängig Wasserstoff oder C₁₋₆-Alkyl ist;
m eine ganze Zahl von 0 bis 4 ist und
n eine ganze Zahl von 0 bis 3 ist.

2. Verbindung nach Anspruch 1, wobei R¹ Triazolyl, Imidazolyl, Tetrazolyl, Pyrazolyl oder Oxadiazolyl ist, die jeweils gegebenenfalls substituiert sind.

3. Verbindung nach Anspruch 2, wobei R¹ [1,2,4]Triazol-1-yl, Imidazol-1-yl, Tetrazol-2-yl, Tetrazol-1-yl, Oxazolidin-1-yl, Tetrazol-5-yl, [1,2,4]Triazol-3-yl, [1,2,3]Triazol-1-yl, [1,2,3]Triazol-2-yl, Imidazol-2-yl, [1,2,4]Triazol-4-yl, Imidazol-4-yl, Pyrazol-1-yl, [1,3,4]Oxadiazol-2-yl, [1,2,4]Oxadiazol-5-yl, Oxazol-5-yl oder [1,2,4]Triazol-2-yl ist, die jeweils gegebenenfalls substituiert sind.

4. Verbindung nach anspruch 3, wobei R¹ 5-Carbamyl-2H-[1.2.4]triazol-1-yl5-Carbamyl 1H-imidazol-1-yl; 3-Carbmethoxyl-1H-[1,2,4]triazol-1-yl; 5-Carbmethoxy-1H-[1,2,4]triazol-1-yl; 5-Carbethoxymethyl-2H-tetrazol-2-yl; 5-Carbethoxymethyl-1H-tetrazol-1-yl; 1-Methan-sulfonylmethyl-1H-tetrazol-5-yl; 1-(2-Hydroxylethyl)-1H-tetrazol-5-yl; 2-(2-Hydroxylethyl)-2H-tetrazol-5-yl; 2-Methansulfonylmethyl-2H-tetrazol-5-yl; 5-Carbmethoxy-1H-imidazol-1-yl; 5-Carbethoxy-1H-imidazol-1-yl; 2H-[1,2,4]Triazol-3-yl; 1H-[1,2,3]Triazol-1-yl; 2H-[1,2,3]Triazol-2-yl; 1H-Tetrazol-5-yl; 2-Methoxymethyl-2H-[1,2,4]triazol-3-yl; 1 -Methyl-1H-tetrazol-5-yl; 1H-Imidazol-2-yl; 1H-Imidazol-1-yl: 4H-[1,2,4]Triazol-4-yl; 1-(2-Hydroxyethyl)-1H-imidazol-2-yl; 3H-1midazol-4-yl; 1H-[1,2,4]Triazol-3-yl; 1-Methyl-1H-tetrazol-5-yl; 2-Methyl-2H-tetrazol-5-yl; 2H-[1,2,4]Triazol-3-yl; 1-(N,N-Dimethylsulfonamid)-1H-imidazol-2-vl; 1H-Pyrazol-1-yl; [1,3,4]Oxadiazol-2-yl; 1H-[1,2,-4]Triazol-1-yl; 1H-Tetrazol-1-yl; 1H-Tetrazol-5-yl; 2H-Tetrazol-2-yl; 3-Methyl-[1,2,4]oxadiazol-5-yi; Oxazol-5-yl; 5-Carbamylmethyl-1H-tetrazol-1-yl; 3-Carbamyl-1H-[1,2,4]triazol-1-yl; 3-Hydroxymethyl-2H-[1,2,4]triazol-2-yl; 3-Carbethoxymethyl-1H-[1,2,4]triazol-1-yl; 3-Carbethoxymethyl-2H-[1,2,4]triazol-2-yl oder 5-(2-Hydroxyethyl)-1H-tetrazol-1-yl ist.

5. Verbindung nach Anspruch 1, wobei Y (CH₂)ₙ ist.

6. Verbindung nach Anspruch 5, wobei n 0 ist.

7. Verbindung nach Anspruch 5, wobei n 1 ist.

8. Verbindung nach Anspruch 5, wobei n 2 ist.

9. Verbindung nach Anspruch 1, wobei Y C=O ist.

10. Verbindung nach Anspruch 1, wobei Y CH(OR³) ist.

11. Verbindung nach anspruch 10, wobei R³ H, Methyl oder Ethyl ist.

12. Verbindung nach Anspruch 1, wobei Y Alkenylen ist.

13. Verbindung nach Anspruch 12, wobei Y Ethenylen ist.

14. Verbindung nach Anspruch 1, wobei R² C₁-₆-Alkyl ist.

15. Verbindung nach Anspruch 14, wobei R² Methyl ist.

16. Verbindung nach Anspruch 1, wobei Ar disubstituiertes Aryl oder disubstituiertes Heteroaryl ist.

17. Verbindung nach Anspruch 16, wobei Ar disubstituiertes Aryl ist.

18. Verbindung nach Anspruch 17. wobei Ar disubstituiertes Phenyl ist.

19. Verbindung nach Anspruch 18, wobei Ar Dihalogenid-substituiertes Phenyl ist und wobei jedes Halogenid unabhängig ausgewählt ist.

20. Verbindung nach Anspruch 19, wobei Ar 2,4-Dichlorphenyl ist.

21. Verwendung einer Verbindung der Formel 1 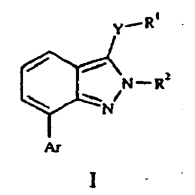 oder eines pharmazeutisch akzeptablen Salzes davon,
worin:
Y (CH₂)ₙ, Alkenylen, CH(OR³) oder C=O ist;
R¹ ein fünfgliedriges Heteroaryl, enthaltend mindestens ein Stickstoffringatom, ist, wobei der fünfgliedrige Heteroarylring gegebenenfalls mit einem oder mehreren Substituenten substituiert ist, von denen jeder unabhängig ausgewählt ist aus der Gruppe, bestehend aus C₁₋₆-Alkyl, C₁-₆-Heteroalkyl, -(CH₂)ₘCOX¹, -(CH₂)ₘSO₂X² und Oxo;
X¹ -OR³ oder -NR⁴R⁵ ist;
X² C₁-C₆ Alkyl oder NR⁴R⁵ ist;
R² Wasserstoff, C₁₋₆-Alkyl, C₁₋₆-Halogenalkyl oder C₃₋₆ Cycloalkyl ist,
Ar Aryl oder Heteroaryl ist, die jeweils gegebenenfalls mit einem oder mehreren Substituenten, unabhängig ausgewählt aus der Gruppe, bestehend aus C₁₋₆-Alkyl, C₁-₆-Alkoxy, C₁-₆-Alkylthio, C₁₋₆-Alkylsulfonyl, -SO₂NR⁴R⁵, Halogen, C₁-₆-Halogenalkyl, Cyano, Nitro und -NR⁶R⁷, substituiert sind;
jeder von R⁶ und R⁷ unabhängig ausgewählt ist aus der Gruppe, bestehend aus Wasserstoff, C₁₋₉-Alky! und C₁₋₉-Alkylcarbonyl;
jeder von R³, R⁴ und R⁵ unabhängig Wasserstoff oder C₁₋₆-Alkyl ist;
m eine ganze Zahl von 0 bis 4 ist und
n eine ganze Zahl von 0 bis 3 ist,
zur Herstellung eines Medikaments zur Vorbeugung oder Behandlung von Störungen, die durch einen positiven allosterischen Modulator eines GABA_{A}-Rezeptors gelindert werden.

22. Verwendung nach Anspruch 21, wobei die Störung Depression, eine Angststörung, eine psychiatrische Störung, eine Lern- oder Wahrnehmungsstörung, eine Schlafstörung, ein Krampf- oder Anfallsleiden oder Schmerz ist.

23. Verwendung nach Anspruch 22, wobei die Verbindung in Kombination mit einem selektiven Serotonin-Wiederaufnahmeinhibitor, einem Corticotropin-releasing-Faktor-Antagonisten oder einem Phosphodiesterase-IV-Inhibitor verabreicht wird.

24. Verwendung nach Anspruch 21, wobei der positive allosterische Modulator eines GABA_{A}-Rezeptors bezogen auf den ocl-Subtyp ein selektiver Modulator der α2-Subtypen ist.

25. Pharmazeutische Zusammensetzung zur Vorbeugung oder Behandlung von Störungen, die von einem positiven allosterischen Modulator eines GABA_{A}-Rezeptors gelindert werden, wobei die Zusammensetzung eine therapeutisch wirksame Menge einer Verbindung nach Anspruch 1 in einem Gemisch mit mindestens einem Verdünnungsmittel, Hilfsmittel oder - Träger umfasst.

## Revendications

1. Composé de formule: 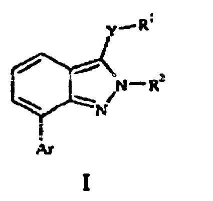 ou un de ses sels pharmaceutiquement acceptables,
où:
Y est (CH₂)ₙ, alcénylène, CH(OR³) ou C=O;
R¹ est un hétéroaryle de 5 chaînons contenant au moins un atome d'azote cyclique, ledit cycle hétéroaryle de 5 chaînons étant éventuellement substitué par un ou plusieurs substituants choisis chacun indépendamment dans le groupe constitué par alkyle en C₁-C₆, hétéroalkyle en C₁-C₆, -(CH₂)ₘCOX¹, -(CH₂)ₘSO₂X², et oxo;
X¹ est -OR³ ou -NR⁴R⁵;
X² est alkyle en C₁-C₆ ou -NR⁴R⁵;
R² est hydrogène, alkyle en C₁-C₆, halogénoalkyle en C₁-C₆ ou cycloalkyle en C₃- C₆;
Ar est aryle ou hétéroaryle, éventuellement substitués chacun par un ou plusieurs substituants choisis indépendamment dans le groupe constitué par alkyle en C₁- C₆, alcoxy en C₁-C₆, alkylthio en C₁-C₆, alkylsulfonyle en C₁-C₆, -SO₂NR⁴R⁵, halogène, halogénoalkyle en C₁-C₆, cyano, nitro et -NR⁶R⁷;
chacun des R⁶ et R⁷ est choisi indépendamment dans le groupe constitué par hydrogène, alkyle en C₁-C₉, et (alkyl en C₁-C₉)carbonyle;
chacun des R³, R⁴ et R⁵ est indépendamment hydrogène ou alkyle en C₁-C₆;
m est un entier de 0 à 4; et
n est un entier de 0 à 3.

2. Composé selon la revendication 1, dans lequel R¹ est triazolyle, imidazolyle, tétrazolyle, pyrazolyle ou oxadiazolyle, chacun d'entre eux étant éventuellement substitué.

3. Composé selon la revendication 2, dans lequel R¹ est [1,2,4]triazol-1-yle, imidazol-1-yle, tétrazol-2-yle, tétrazol-1-yle, oxazolidin-1-yle, tétrazol-5-yle, [1,2,4]triazol-3-yle, [1,2,3,]triazol-1-yle, [1,2,3,]triazol-2-yle, imidazol-2-yle, [1,2,4]triazol-4-yle, imidazol-4-yle, pyrazol-1-yle, [1,3,4]oxadiazol-2-yle, [1,2,4]oxadiazol-5-yle, oxazol-5-yle, ou [1,2,4]triazol-2-yle, chacun d'entre eux étant éventuellement substitué.

4. Composé selon la revendication 3, dans lequel R¹ est 5-carbamyl-2H-[1.2,4]trazol-1-yle; 5-carbarmyl-1H-imidazol-1-yle; 3 carbométhoxy 1H-[1,2,4]triazol-1-yle; 5-carbométhoxy-1H-[1,2,4]triazol-1-yle; 5-carbéthoxyméthyl-2H-tétrazol-2-yle; 5-carbéthoxyméthyl-1H-tétrazol-1-yle; 1-méthanesulfonylméthyl-1 H-tétrazol-5-yle; 1-(2-hydroxyéthyl)- 1-tétrazol-5-yle; 2-(2-hydroxyéthyl)-2H-tétrazol-5-yle; 2-méthanesulfonylméthyl-2H-tétrazol-5-yle; 5-carbométhoxy-1H-imidazol-1-yle; 5-carbéthoxy-1H-imidazol-1-yle; 2H-[1,2,4]triazol-3-yle; 1H-[1,2,3]triazol-1-yle; 2H-[1,2,3]triazol-2-yle; 1H-tétrazol-5-yle; 2-méthoxyméthyl-2H-[1,2,4]triazol-3-yle; 1-méthyl-1H-tétrazol-5-yle; 1H-imidazol-2-yle; 1-imidazol-1-yle; 4H-[1,2,4]triazol-4-yle; 1-(2-hydroxyéthyl)-1H-imidazol-2-yle; 3H-imidazol-4-yle; 1H-[1,2,4]triazol-3-yle; 1-méthyl-1H-tétrazol-5-yle; 2-méthyl-2H-tétrazol-5-yle; 2H-[1,2,4]triazol-3-yle; 1-(N,N-diméthylsulfonamide)-1H-imidazol-2-yle; 1H-pyrazol-1-yle; [1,3,4]-oxadiazol-2-yle; 1H-[1,2,4]triazol-1-yle; 1H-tétrazol-1-yle; 1H-tétrazol-5-yle; 2H-tétrazol-2-yle; 3-méthyl-[1,2,4]oxadiazol-5-yle; oxazol-5-yle; 5-carbamylméthyl-1H-tétrazol-1-yle; 3-carbamyl- H-[1 1,2,4]triazol-1-yle; 3-hydroxyméthyl-2H-[1,2,4]triazol-2-yle; 3-carbéthoxyméthyl-1H-[1,2,4]triazol-1-yle; 3-carbéthoxyméthyl-2H-[1,2,4]triazol-2-yle; ou 5-(2-hydroxyéthyl)-1H-tétrazol-1-yle.

5. Composé selon la revendication 1, dans lequel Y est (CH₂)ₙ.

6. Composé selon la revendication 5, dans lequel n est 0.

7. Composé selon la revendication 5, dans lequel n est 1.

8. Composé selon la revendication 5, dans lequel n est 2.

9. Composé selon la revendication 1, dans lequel Y est C=O.

10. Composé selon la revendication 1, dans lequel Y est CH(OR³).

11. Composé selon la revendication 10, dans lequel R³ est H, méthyle ou éthyle.

12. Composé selon la revendication 1, dans lequel Y est alcénylène.

13. Composé selon la revendication 12, dans lequel Y est éthénylène.

14. Composés selon la revendication 1, dans lequel R² est alkyle en C₁-C₆.

15. Composé selon la revendication 14, dans lequel R² est méthyle.

16. Composé selon la revendication 1, dans lequel Ar est aryle disubstitué ou hétéroaryle disubstitué.

17. Composé selon la revendication 16, dans lequel Ar est aryle disubstitué.

18. Composé selon la revendication 17, dans lequel Ar est phényle disubstitué.

19. Composé selon la revendication 18. dans lequel Ar est phényle substitué par deux halogènes, et chaque halogène est choisi de façon indépendante.

20. Composé selon la revendication 19, dans lequel Ar est 2,4-dichlorophényle.

21. Utilisation d'un composé de formule I 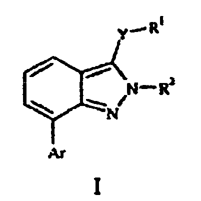 ou d'un de ses sels pharmaceutiquement acceptables,
où:
Y est (CH₂)ₙ, alcénylène, CH(OR³) ou C-O;
R¹ est un hétéroaryle de 5 chaînons contenant au moins un atome d'azote cyclique, ledit cycle hétéroaryle de 5 chaînons étant éventuellement substitué par un ou plusieurs substituants choisis chacun indépendamment dans le groupe constitué par alkyle en C₁-C₆, hétéroalkyle en C₁-C₆, -(CH₂)ₘCOX¹, -(CH₂)ₘSO₂X², et oxo;
X¹ est -OR³ ou -NR⁴R⁵;
X² est alkyle en C₁-C₆ ou -NR⁴R⁵;
R² est hydrogène, alkyle en C₁-C₆, halogénoalkyle en C₁-C₆ ou cycloalkyle en C₃- C₆;
Ar est aryle ou hétéroaryle, éventuellement substitués chacun par un ou plusieurs substituants choisis indépendamment dans le groupe constitué par alkyle en C₁- C₆, alcoxy en C₁-C₆, alkylthio en C₁-C₆, alkylsulfonyle en C₁-C₆, -SO₂NR⁴R⁵, halogène, halogénoalkyle en C₁-C₆, cyano, nitro et -NR⁶R⁷;
chacun des R⁶ et R⁷ est choisi indépendamment dans le groupe constitué par hydrogène, alkyle en C₁-C₉, et (alkyl en C₁-C₉)carbonyle;
chacun des R³, R⁴ et R⁵ est indépendamment hydrogène ou alkyle en C₁-C₆;
m est un entier de 0 à 4; et
n est un entier de 0 à 3
pour la préparation d'un médicament destiné à la prévention ou au traitement de troubles soulagés par un modulateur allostérique positif d'un récepteur GABA,4.

22. Utilisation selon la revendication 21. dans laquelle ledit trouble est une dépression, un trouble d'anxiété, un trouble psychiatrique, un trouble de l'apprentissage ou un trouble cognitif, un trouble du sommeil, un trouble convulsif ou épileptique ou une douleur.

23. Utilisation selon la revendication 22, dans laquelle ledit composé est administré en combinaison avec un inhibiteur sélectif de la recapture de la sérotonine, un antagoniste du facteur de libération de la corticotropine ou un inhibiteur de la phosphodiestérase IV.

24. Utilisation selon la revendication 21, dans laquelle ledit modulateur allostérique positif d'un récepteur de GABA_{A} est un modulateur sélectif des sous-types α2 par rapport au sous-type al.

25. Composition pharmaceutique pour la prévention ou le traitement de troubles soulagés par un modulateur allostérique positif d'un récepteur GABA_{A}, ladite composition comprenant une quantité thérapeutiquement efficace d'un composé selon la revendication 1 en mélange avec au moins un diluant, excipient ou véhicule.
